# EUROPEAN PATENT SPECIFICATION

(11) **EP 4 161 580 B1**
(45) Date of publication and mention of the grant of the patent: **18.03.2026**
(21) Application number: 21818048.7
(22) Date of filing: 04.06.2021
(51) Int. Cl.: A61K 47/61, A61K 36/185, A61P 17/00, A61P 27/06, C08B 37/08

(54) **CANNABINOID-HYALURONIC ACID BIOCONJUGATES**
BIOKONJUGATE VON CANNABINOID-HYALURONSÄURE
BIOCONJUGUÉS CANNABINOÏDE-ACIDE HYALURONIQUE

(30) Priority: 05.06.2020 US 202063035017 P
(43) Date of publication of application: 12.04.2023
(73) Proprietor: London Pharmaceuticals and Research Corporation, London, ON N6G 0K4 (CA)
(72) Inventor: LAWENDY, Abdel-rahman, London, Ontario N6G 0K4 (CA); ALASSUITY, Ahmed Said, London, Ontario N6G 4X5 (CA)
(74) Representative: Zeuner Summerer Stütz
(86) International application number: PCT/CA2021/050769
(87) International publication number: WO 2021/243467

(56) References cited:
- WO-A1-2009/018389
- WO-A1-2009/018389
- WO-A1-2019/159168
- WO-A1-2020/263888
- US-A1- 2013 184 354
- TIAN CIHUI ET AL: "Tween 80-modified hyaluronic acid-ss-curcumin micelles for targeting glioma: Synthesis, characterization and their in vitro evaluation", vol. 120, 1 December 2018 (2018-12-01), NL, pages 2579 - 2588, XP093145900, ISSN: 0141-8130, Retrieved from the Internet <URL:https://www.sciencedirect.com/science/article/pii/S0141813018332458/pdfft?md5=0fd47b25fa508696dc745de0dd1a680b&pid=1-s2.0-S0141813018332458-main.pdf> DOI: 10.1016/j.ijbiomac.2018.09.034
- CAROLE E SCHANT ET AL: "Chemical modifications of hyaluronic acid for the synthesis of derivatives for a broad range of biomedical applications", CARBOHYDRATE POLYMERS, APPLIED SCIENCE PUBLISHERS , LTD BARKING, GB, vol. 85, no. 3, 11 March 2011 (2011-03-11), pages 469 - 489, XP028383766, ISSN: 0144-8617, [retrieved on 20110321], DOI: 10.1016/J.CARBPOL.2011.03.019

## Description

### Field of the Invention

The present invention relates to new solubilized form of cannabinoids, in particular, to cannabinoid-hyaluronic acid bioconjugates.

### Background

A large number of reports in the scientific literature confirm the health benefits and the biological activities of many types of phytochemicals. Cannabinoids are a unique class of phytochemicals produced naturally by the plants, such as *Cannabis sativa, Cannabis indica, Cannabis ruderalis,* and other related plant species.

Endocannabinoids can be found naturally inside human bodies. However, only a few endogenous cannabinoids, which are endogenous lipids that bind to cannabinoids receptors, have been detected and isolated, such as anandamide and 2-arachidonylglycerol.

Many scientific reports have confirmed the pharmacologically beneficial effects of cannabinoids. Cannabinoids are being investigated for therapeutic uses, such as anti-inflammatory, stimulation of appetite (especially with cancer patients), analgesia, muscle relaxation, and for treatment of nausea, vomiting, arthritis, seizures, and many other diseases.

Due to the scientifically proven adverse health effects of smoking cannabis and other plant materials, there is a need for a smokeless alternative to obtain the health benefits of cannabis without the adverse effects of smoking. Edible cannabis products are expected to lead the market due to the convenience of use, unlike other formulations such as injections or patches.

Hyaluronic acid (HA, hyaluronan) is a water-soluble natural polysaccharide, which is ubiquitous in nature, it can be found widely in the extracellular matrix of tissues in higher organisms. Hyaluronan plays vital roles in the binding of other matrix components. Hyaluronan or its sodium salt (hyaluronate) can be isolated from rooster combs. Some strains of bacteria such as *Staphylococcus zooepidemicus* and *Streptococcus equi* can also produce HA through fermentation.

Hyaluronan is a natural unbranched (linear) high molecular weight biopolymer. The molecular weight is usually in the range of 10⁵ - 10⁷ Da, however, lower average molecular weight can also be obtained starting from as low as 1200 Da. Hyaluronan has a unique chemical structure consisting of alternating units (monomers) of β-(1-4) N-acetyl-D-glucosamine and β-(1-3) D-glucuronic acid, as illustrated in the structural formula, below.

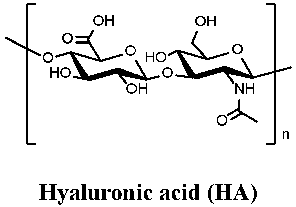

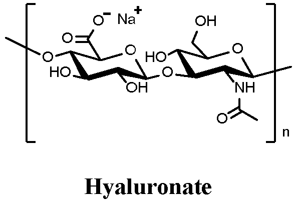

Due to the high molecular weight of hyaluronan, its aqueous solution has high viscosity which provides synovial fluid viscoelasticity and lubricating effect. Hyaluronan also has many other physiological functions (such as modulating inflammation, angiogenesis, and tissue lubrication) and therapeutic applications such as wound healing, antiaging, and many other applications.

Due to the lipophilic nature of cannabinoids, their use in edible products is limited. In addition, the bioavailability of cannabinoids is very low. It is estimated that about 90% of ingested cannabinoids, in edible formulations, will not reach the biological target.

Sayre et al, US Pat. No. 2019/0316144 A1, discloses water soluble cannabinoids produced by genetically modified plant cells in suspension cultures. This approach is not favorable to many consumers as there are growing concerns about the safety and possible negative health effects related to genetically modified food products. Studies have shown that when consumers are informed about possible negative health impact of genetically modified products, the willingness to buy the products drops significantly. From a production point of view the stability of the gene is a concern. Sayre et al, US Pat. No. 2019/0078168 A1, uses a similar approach to produce soluble cannabinoids in yeast.

Silver R., US Pat. No. 2019/0060227 A1, discloses water soluble cannabinoids (and cannabis oil) using emulsifiers. This product is complex to formulate as it requires multiple components. On the other hand, thermodynamically the emulsion is considered an unstable system as the emulsion of two immiscible liquids has a tendency to separate to reduce the interfacial energy. There are several reported phenomena which can contribute to the destabilization of emulsions, such as coalescence, sedimentation, creaming, flocculation, and phase inversion. Galli and Rigassi, WO2006/094829 A1, discloses a similar microemulsion approach to produce dispersible cannabinoids compositions.

Degeeter and Johnson, WO2017/183011 A1, discloses the use of cyclodextrins to produce water soluble cannabinoids through inclusion complexes. Cyclodextrins (CD) are cyclic oligosaccharides which can cause reversible diarrhoea, cecal enlargement, and nephrotoxic effects in animals, and may cause diarrhoea in humans at high dose. The stability of drugs, such as cannabinoids, may be negatively affected in the inclusion complex with CDs as some drugs undergo CD catalyzed degradation under certain conditions.

WO2009/018389 A1 discloses prodrugs of CBD including CBD-HA. Derivatization of HA in this patent occurs exclusively via its CH₂OH functionality. In addition, several academic publications disclosed several synthetic routes for the preparation of HA derivatives (Bioconjugate Chemistry, 1991, 2, 232-241; Carbohydr. Polym. 2011, 85, 469-489). These routes usually require multiple and complex steps resulting in higher cost of production and the compounds are isolated in low yields or as isomers with poor control on derivatization and drug loading capacity (LC%). In many cases, no biological data or insufficient information are provided about their activities.

The paper Tian Cihui, et al., "Tween 80-modified hyaluronic acid-ss-curcumin micelles for targeting glioma: Synthesis, characterization and their in vitro evaluation", INTERNATIONAL JOURNAL OF BIOLOGICAL MACROMOLECULES, vol. 120, 1 December 2018, pages 2579 - 2588, discloses a conjugate with a backbone of hyaluronic acid and curcumin, attached by way of a spacer. Document US 2013/184354 A1 discloses Silicone and Hylauronic Acid (HLA) Delivery Systems for Products by Sustainable Processes for Medical Uses Including Wound Management. Accordingly, there is a need for water soluble cannabinoids with improved pharmacokinetic/pharmacodynamic profile for both food and medicinal applications.

The cannabinoid-HA bioconjugates, according to the present invention, address the main challenges facing cannabinoid formulations, particularly topical, injectable, and oral (such as edible and energy drinks). Embodiments of the cannabinoid-HA conjugates improve the aqueous solubility, taste, stability, and bioavailability of cannabinoids.

### Summary of the Invention

The cannabinoid-HA conjugates, according to the present invention, provide an oral delivery system for cannabinoids and synergistic compounds for the treatment of inflammation and pain with an improved pharmacokinetic/pharmacodynamic profile, compared to some other forms of oral delivery of cannabinoids.

The invention provides the cannabinoid-HA conjugate of claim 1 and the compounds of claims 13 and 29.

In some embodiments, the spacer is selected from the group consisting of L1, L2, L3, and L4: 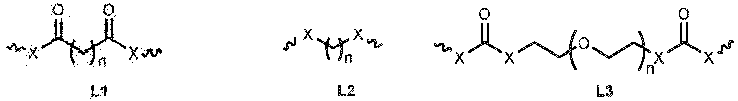 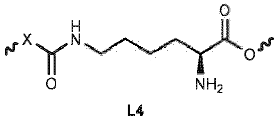 wherein, x = S, O, or NH; and, for L1 and L2, n = 1-10, and for L3, n = 1-40.

In some embodiments, the cannabinoid-HA conjugate has one or more amide-based spacers for sustained release of the cannabinoid and one or more ester-based spacers for quick release of the cannabinoid.

In some embodiments, a synergistic compound is attached by way of a spacer to the one or more derivatization sites of the polysaccharide backbone.

.

### Detailed Description of the Invention

A cannabinoid-HA conjugate, according to the present invention, has a hyaluronic acid or an analog or derivative thereof forming a polysaccharide backbone, with one or more derivatization sites on the polysaccharide backbone or the peripheral groups attached to the polysaccharide backbone. At least one of the one or more derivatization sites are conjugated directly or indirectly with a cannabinoid.

In some embodiments, the cannabinoid-HA conjugate has polysaccharide backbone of hyaluronic acid or an analog or derivative of hyaluronic acid having one or more derivatization sites selected from the group of function groups consisting of: primary hydroxyl groups, secondary hydroxyl groups, carboxyl groups, acetamido groups, and the reducing end of the polysaccharide. At least one of the one or more derivatization sites are conjugated directly or indirectly with a cannabinoid.

Unlike prior cannabinoid conjugates, the present invention utilizes more efficient chemistry with higher drug loading capacity (LC%) and wider possibilities of generating unique cannabinoid-HA derivatives. Furthermore, the novel cross-linking approach of some embodiments provides optimum control over the physical and mechanical properties, in particular the hydration and gelation capacity of the cannabinoid-HA conjugates.

In addition, in some embodiments, the conjugate may be formed by way of a covalent connection between a cannabinoid and the N-acetyl group (-NHCOCH₃) of HA. This chemical modification would have negligible effect on hydration capacity and gelation properties of the HA, compared to conjugation at other derivatization sites, such as the carboxyl group.

The present invention discloses novel cannabinoid-HA bioconjugates, belonging to the following general and illustrative formulas I and II:

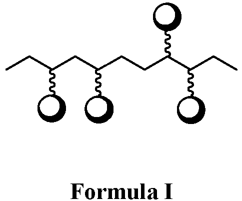

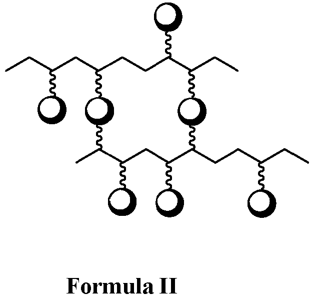

In formulas I and II, above, the 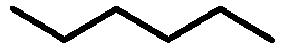 is polysaccharide backbone of hyaluronic acid or an analog or a derivative thereof. The 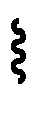 is a biocompatible spacer (or linker) (L), independently attached to one or more derivatization sites selected from the group consisting of: hydroxyl (-OH), acetamido (-NHCOCH₃), deacylated amino (-NH₂), and carboxylic acid (-COOH) functional groups of HA backbone. The 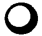 is independently selected from cannabinoids (Cann) or one or more synergistic compounds.

In some embodiments, one or more synergistic compounds are conjugated to the hyaluronic acid. The synergistic compounds can have a synergistic or additive effect with the cannabinoids, or they can affect different targets or receptors in order to broaden the pharmacological effect of the conjugate.

In some other embodiments, cannabinoids are used as a biocompatible and biologically active cross-linking agent to covalently cross link the HA backbone in order to minimize its biodegradation, increase duration of action, and improve stability. One disadvantage of cross-linking the HA backbone is that it is known to cause an inflammatory response. Preferably, a cannabinoid is used for cross-linking. Preferably, CBD is used as the biologically compatible and active cross-linker. Using CBD or another similar cannabinoid, which has an anti-inflammatory effect, as a cross-linker for the HA backbone helps to overcome the disadvantage of cross-linking the HA backbone in terms of its effect on the inflammatory response. This permits the degree of cross-linking to be used to select for the desired drug release time and duration of action, since higher cross-linking results in a reduction in the speed of degradation in the body.

In some other embodiments, either or both covalent cross-linking and physical cross linking are used to optimize the physical and biological properties of HA. The degree of covalent crosslinking may be 5%, 10%, 20%, 30%, 40, or 50% (relative to the available groups for crosslinking). Preferably, the degree of cross-linking is between 20% and 40%.

In some other embodiments, the ratio of covalent cross-linking relative to physical cross linking is 1:1, 1:2, 1:3, 1:4, 1:5, 1: 10, 1:20. Preferably, the ratio of covalent cross linking relative to physical cross linking is 1:5 or 1:10 or 1:20.

In some embodiments, the present invention comprises a pharmaceutical composition comprising a compound disclosed herein, or a pharmaceutically acceptable salt thereof, together with one or more pharmaceutically acceptable diluents, and excipients. The pharmaceutical composition may also include a compound disclosed herein, in combination with one or more other therapeutically active compounds by the same or different mode of action.

In some embodiments, the present invention comprises a method of administering a therapeutically effective amount of a compound disclosed herein for the general purpose of improving health and wellness or specific treatment of a minor ailment and a disease condition of patients such as pain, inflammation, and arthritis, together with one or more pharmaceutically acceptable diluents, and excipients. The delivery may be topical or systemic.

In some embodiments, the present invention comprises a method of administering a therapeutically effective amount of a compound disclosed herein, in combination with one or more therapeutically effective compounds by the same or different mode of action, together with one or more pharmaceutically acceptable diluents, and excipients, for the purpose of improving health and wellness or specific treatment of a minor ailment or a disease condition of a patient such as pain, inflammation or arthritis. The delivery may be topical or systemic.

In some embodiments, cannabinoids are conjugated to HA or an analog or a derivative of such compounds such as chondroitin sulfate, chitosan, polyacrylic acid carboxypolymethylene, carboxymethylcellulose and the like.

In some embodiments, the spacers (or linkers) "L", independently, may be O, a linear or branched, substituted or unsubstituted alkyl chain, substituted or unsubstituted unsaturated carbon chain, C=O, O=C-C=O, O=C-(CH₂)nC=O, where n is any integer number, O=C-(CR₂)nC=O, where R is independently any substitution such as H, alkyl, halide, CN, NH2, OH, COOH, and the like, NH-CO, NH-alkyl, substituted or unsubstituted alkyl-C=O, substituted or unsubstituted alkyl-NH-C=O, substituted or unsubstituted alkenyl-C=O, substituted or unsubstituted alkenyl -C=O, substituted or unsubstituted alkenyl-NH-C=O, substituted or unsubstituted alkenyl-C=O, substituted or unsubstituted alkynyl-C=O, substituted or unsubstituted alkynyl -C=O, substituted or unsubstituted alkenyl-NH-C=O, substituted or unsubstituted alkynyl-C=O, cycloalkyl, or cycloalkenyl, or heterocyclic structures, preferably azole derivatives, such as triazole, which may be optionally substituted with alkyl, carbonyl, or alkoxy moieties, and the like.

Certain preferred spacers of the present invention include the following examples:

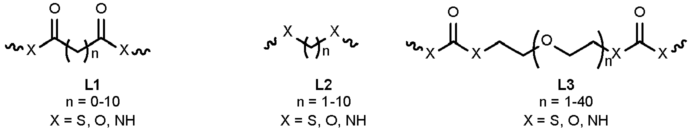

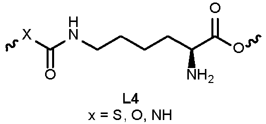

In some embodiments, "Cann" is any cannabinoid, which can be independently chosen from the following group: delta-9-tetrahydrocannabinol (THC), cannabidiol (CBD), cannabinol (CBN), cannabinolic acid (CBNA), cannabigerol (CBG), cannabigerol (CBG), cannabigerovarin (CBGV), cannabichromene (CBC), cannabicyclol (CBL), canabivarol (CBV), tetrahydrocannabivarin (THCV), cannabidivarin (CBDV), cannabichromevarin (CBCV), cannabigerol monoethyl ether (CBGM), cannabigerolic acid monoethyl ether (CBGAM) cannabidiolic acid (CBDA), cannabigerovarinic (CBGVA), cannabichromenic acid (CBCA), cannabichromenic acid (CBCA), cannabidiol monomethylether (CBDM), cannabidiol-C₄ (CBD-C₄), cannabidivarinic (CBDVA), cannabidiorcol (CBD-C₁), delta-9-tetrahydrocannabinolic acid A (THCA-A), delta-9-tetrahydrocannabinolic acid B (THCA-B), delta-9-tetrahydrocannabinolic acid-C₄ (THCA-C₄), delta-8-tetrahydrocannabinolic acid (delta-8-THCA), delta-8-tetrahydrocannabinol (delta-8-THC), delta-9-tetrahydrocannabinol-C₄ (THC-C₄), delta-9-tetrahydrocannabiorcolic acid (THCA-C₁), delta-9-tetrahydrocannabiorcol-C₁ (THC-C₁), tetrahydrocannabivarinic acid (THCVA), cannabicycolic acid (CBLA), cannbicyclol (CBL), cannabicyclovarin (CBLV), cannabielsoic acid A (CBEA-A), cannabielsoic acid B (CBEA-B), cannabielsoin (CBE), cannabivarin, cannabinol-C₄ (CBN-C₄), cannabinol methylether (CBNM), cannabiorcol (CBN-C₁), cannabinol-C₂ (CBN-C₂), cannabinodiol (CBND), cannabinodivarin (CBVD), cannabitriol (CBT), cannabitriolvarin (CBTV), dehydrocannabifuran (DCBF), cannabifuran, cannabicitran (CBT), cannabiripsol (CBR), '11-hydroxytetrahydrocannabinol' (11-OH-THC), '11-nor-9-carboxy-tetrahydrocannabinol' (THC-COOH), or any derivatives or analogues of the above mentioned cannabinoids, any other similar compound which is isolated from plants, or mixtures or combinations thereof.

In some embodiments, two or more different cannabinoids are conjugated to the HA backbone. The cannabinoid is conjugated directly or indirectly through a spacer to one, two or more of the derivatization sites.

In some other embodiments, the drug loading capacity (LC%) is 10, 20, 30, 40, 50, 60, 80% (w/w). Preferably, the LC% is 20, 30, 40, 50%. More preferably, the LC% is 30-40%.

In some embodiment, one or more synergistic compounds are conjugated to the hyaluronic acid. The synergistic compounds can have a synergistic effect with the cannabinoids, or they can affect different targets or receptors in order to broaden the pharmacological effect of the conjugate.

In some embodiments, the synergistic compounds are another therapeutic agent (e.g., NSAID such as diclofenac sodium), functional fatty acids (FA) such as oleyl, stearyl, linolenoyl, palmitoyl, caproyl, arachidonoyl, Anandamide, phosphatidylethanolamine or the like, or amino acids (AA) such as leucine, isoleucine, lysine, valine and the like, oligosaccharide and sugars such as glucosamine, vitamins such as vit C and the like. These synergistic compounds preferably provide synergistic anti-inflammatory or antiaging effects, or both.

In some embodiments, the cannabinoid-HA bioconjugates provide cannabinoids in a water-soluble form through the bioconjugation of one or more cannabinoids with hyaluronic acid (hyaluronan). A water-soluble cannabinoid-HA conjugate significantly increases the stability and bioavailability of cannabinoids. In one preferred example, the conjugates increase the stability of CBD-A by conjugating the chemically labile carboxylic acid of CBDA to the HA backbone.

In some embodiments, the compounds of the present invention are suitable for any cosmetic, pharmaceutical or nutraceutical application such as, but not limited to, all skin-care conditions, ocular applications, or joint disorders.

In another embodiment, the cannabinoid conjugates according to the present invention can be used in multiple different formulations such as oral delivery systems, transdermal drug delivery systems, ocular drug delivery systems, and other delivery systems which can be used to treat disease such as eye diseases, stomach and intestine diseases, skin diseases, nervous systems diseases, pain and inflammation diseases, muscle and knee pain, and cancer.

In some embodiments, the cannabinoid-HA conjugates provides both short-term quick release of cannabinoids and long-term sustained release of cannabinoids based on degree of crosslinking and the spacers used. This can be achieved by double conjugating the hyaluronan and by selection of the cleavable spacer between the hyaluronan and the cannabinoids. Preferably, long-acting conjugates are obtained by using amide-based spacers with a high degree of covalent conjugation. Preferably, short-acting conjugates are obtained by using ester-based spacers with a low degree of covalent conjugation. Amide-based spacers break down after about 12 hours in the body, while ester-based spacers break down after about 2 hours in the body. This is useful in the treatment of inflammation and pain to quickly alleviate the symptoms and also provide longlasting relief to the patient.

Certain preferred embodiments of the present invention include the following examples:

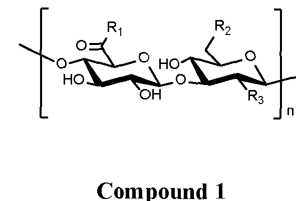

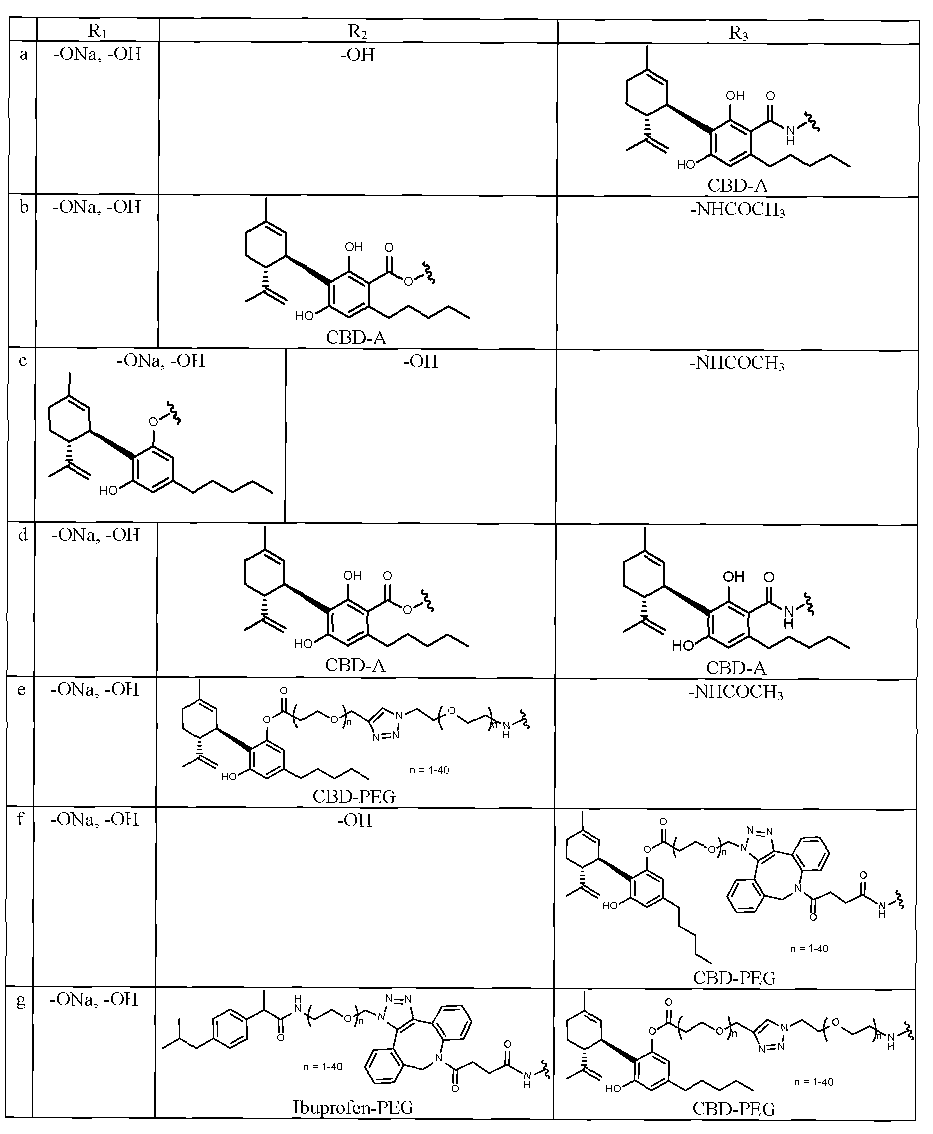

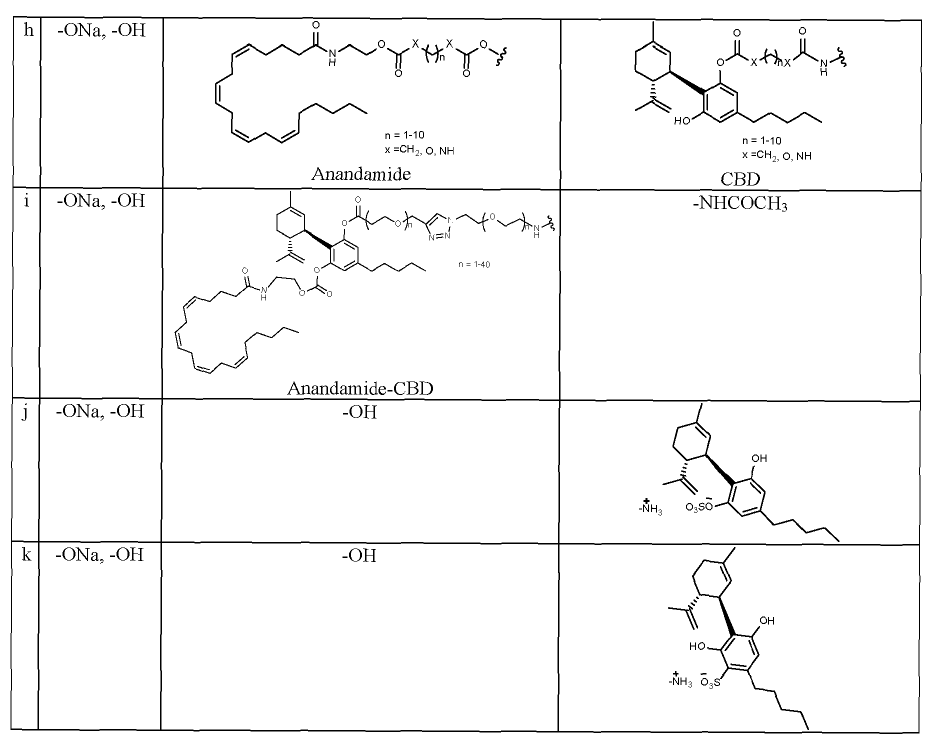

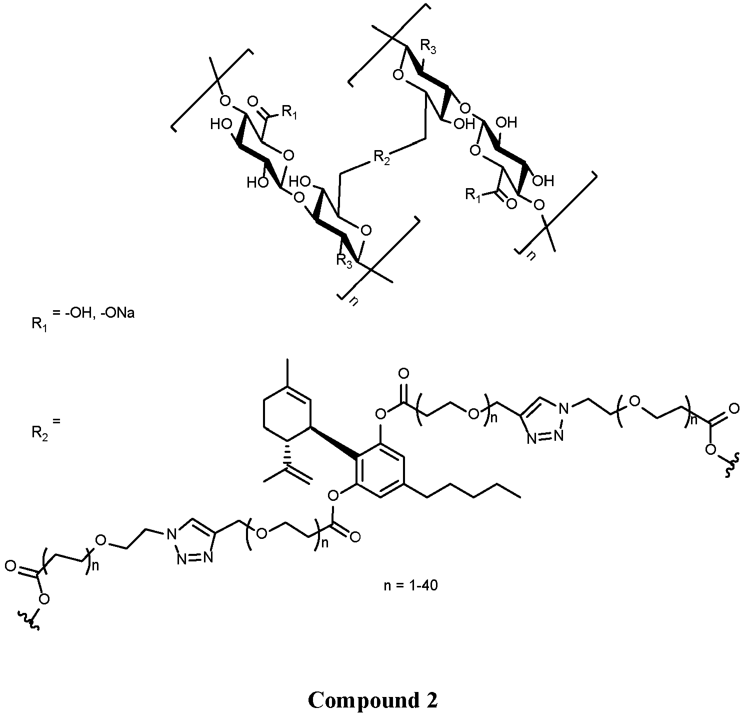

| | R₃ |
|---|---|
| a | 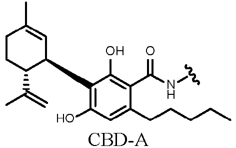 |
| b | 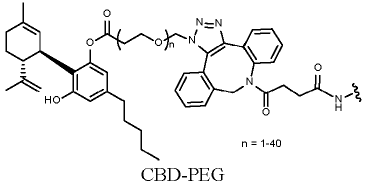 |
| c | 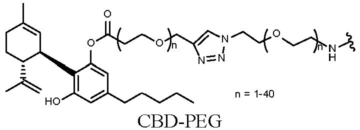 |
| d | 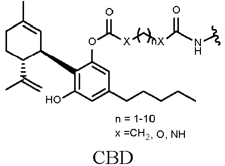 |
| e | 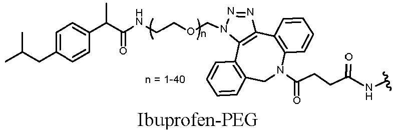 |
| f | 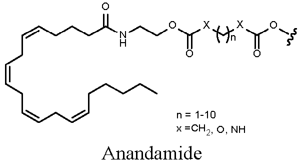 |
| g | 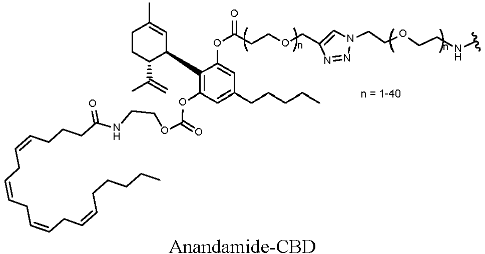 |

Unless otherwise defined, all technical and scientific terms used herein have the same meaning as commonly understood by one of ordinary skill in the art to which this invention belongs. As used in the description of the invention and the appended claims, the singular forms "a," "an," and "the" are intended to include the plural forms as well, unless the context clearly indicates otherwise.

Unless otherwise indicated, all numbers used in the specification and claims are to be understood as being modified in all instances by the term "about." Accordingly, unless indicated to the contrary, the numerical parameters set forth in the specification included in this invention and the claims are approximations that may vary depending upon the desired properties sought to be obtained by the present invention. At the very least, each numerical parameter should be construed in light of the number of significant digits and ordinary rounding approaches.

Although that the numerical ranges and parameters setting forth the broad scope of the invention are approximations, the numerical values set forth in the specific examples are reported to the best of the inventors' knowledge. Any numerical value; however, inherently contains certain errors necessarily resulting from the standard deviation found in their respective testing measurements. Every numerical range given throughout this specification will include every narrower numerical range that falls within such broader numerical range, as if such narrower numerical ranges were all expressly written herein.

**In** the present disclosure the term "about" can allow for a degree of variability in a value or range, for example, within 1%, within 5%, or within 10% of a stated value or of a stated limit of a range. **In** the present disclosure the term "substantially" can allow for a degree of variability in a value or range, for example, within 90%, within 95%, 99 %, 99.5%, 99.9%, 99.99%, or at least about 99.999% or more of a stated value or of a stated limit of a range.

As used herein, the terms "comprising", "including", "having" and grammatical variants thereof are to be taken as specifying the stated features, integers, steps or components but do not preclude the addition of one or more additional features, integers, steps, components or groups thereof.

As used herein, the following terms and phrases shall have the meanings set forth below. Unless defined otherwise, all technical and scientific terms used herein have the same meaning as commonly understood to one of ordinary skill in the art.

The terms "hyaluronan" or "HA" in the present disclosure refer to hyaluronic acid, or its salt hyaluronate and also include any natural or synthetic form or derivative of hyaluronan and any analogues which preserve the ability (completely or partially) of the molecule to bind to HA binding proteins or receptors.

The term "soluble" or "solubilized" refers to any compound which is capable of forming a substantially homogenous mixture that remains stable enough to function as a solution. As used herein, the term is not restricted to the traditional definition of a solute dissolving in a solvent to form a solution. It also includes any pseudo solution and any micro or nano systems which can be produced, processed, or consumed in aqueous systems.

Unless indicated otherwise, the term "cannabinoid" as used herein refers to a compound that affects the endocannabinoid system or otherwise act on the cannabinoid receptors of cells in a patient. Cannabinoids are agonists or antagonists or allosteric modulators to receptors in the endocannabinoid system. They include phytocannabinoids, such as those found in *Cannabis sativa* and other plants, and synthetic cannabinoids. In particular, they include delta-9-tetrahydrocannabinol (THC), cannabidiol (CBD), cannabinol (CBN), cannabinolic acid (CBNA), cannabigerol (CBG), cannabigerol (CBG), cannabigerovarin (CBGV), cannabichromene (CBC), cannabicyclol (CBL), canabivarol (CBV), tetrahydrocannabivarin (THCV), cannabidivarin (CBDV), cannabichromevarin (CBCV), cannabigerol monoethyl ether (CBGM), cannabigerolic acid monoethyl ether (CBGAM) cannabidiolic acid (CBDA), cannabigerovarinic (CBGVA), cannabichromenic acid (CBCA), cannabichromenic acid (CBCA), cannabidiol monomethylether (CBDM), cannabidiol-C₄ (CBD-C₄), cannabidivarinic (CBDVA), cannabidiorcol (CBD-C₁), delta-9-tetrahydrocannabinolic acid A (THCA-A), delta-9-tetrahydrocannabinolic acid B (THCA-B), delta-9-tetrahydrocannabinolic acid-C₄ (THCA-C₄), delta-8-tetrahydrocannabinolic acid (delta-8-THCA), delta-8-tetrahydrocannabinol (delta-8-THC), delta-9-tetrahydrocannabinol-C₄ (THC-C₄), delta-9-tetrahydrocannabiorcolic acid (THCA-C₁), delta-9-tetrahydrocannabiorcol-C₁ (THC-C₁), tetrahydrocannabivarinic acid (THCVA), cannabicycolic acid (CBLA), cannbicyclol (CBL), cannabicyclovarin (CBLV), cannabielsoic acid A (CBEA-A), cannabielsoic acid B (CBEA-B), cannabielsoin (CBE), cannabivarin, cannabinol-C₄ (CBN-C₄), cannabinol methylether (CBNM), cannabiorcol (CBN-C₁), cannabinol-C₂ (CBN-C₂), cannabinodiol (CBND), cannabinodivarin (CBVD), cannabitriol (CBT), cannabitriolvarin (CBTV), dehydrocannabifuran (DCBF), cannabifuran, cannabicitran (CBT), cannabiripsol (CBR), '11-hydroxytetrahydrocannabinol' (11-OH-THC), '11-nor-9-carboxy-tetrahydrocannabinol' (THC-COOH), or any derivatives or analogues of the above mentioned cannabinoids, any other similar compounds which are isolated from plants, or mixtures or combinations thereof.

As used herein, the term "CBD" refers to (cannabidiol) unless indicated otherwise. The term "CBD-A" refers to cannabidiol carboxylic acid unless indicated otherwise.

The term "extract" in the present disclosure refers to compounds from plants that have been extracted and concentrated using one of the many known extraction methods, including hydrocarbon and non-hydrocarbon solvent extracts, maceration, percolation, decoction, reflux extraction, Soxhlet extraction, pressure liquid extraction, supercritical fluid extraction, ultrasonic assisted extraction, microwave assisted extraction, pulsed electrical field extraction, enzyme assisted extraction, hydro distillation and steam distillation, affinity extraction or other extraction methods, or combination of methods known to those skilled in this field.

The term "patient" includes human and non-human animals such as companion animals, including horses, dogs, cats and the like, and livestock animals. Livestock animals are animals raised for production of food, textiles, or other animal-based products. The patient to be treated is preferably a mammal, in particular a human being.

The terms "spacer" or "linker" refers to any chemical backbone, or chemical molecule, which has one, two, or multiple functional groups, which may be used to conjugate or cross-link chemical molecules which may be used to link two molecules together or to conjugate or cross-link chemical molecules, and may be used alone or in mixtures or combinations thereof.

The terms "pharmaceutically acceptable diluent" and "pharmaceutically acceptable excipient" are art-recognized and refer to a pharmaceutically acceptable material, composition or vehicle, such as a liquid or solid filler, solvent or encapsulating material, involved in carrying or transporting any subject composition or component thereof. Each such compound must be "acceptable" in the sense of being compatible with the subject composition and its components and not injurious to the patient. Some examples of materials which may serve as pharmaceutically acceptable diluents or excipients include: sugars, such as lactose and maltose; starches, such as corn starch and gelatinized starch; cellulose, and its derivatives, such as carboxymethyl cellulose salt, and hydroxypropylmethyl cellulose; thickening agents such as gelatin and tragacanth; disintegrants such as copovidone; other excipients, such as cocoa butter and suppository waxes and pyrogen-free water for sterile products; and other non-toxic compatible substances employed in pharmaceutical formulations.

As used herein, the term "administering" includes all means of introducing the compositions described herein to the subject, including mainly topical administering to any part of the subject. The compositions described herein may be administered in unit dosage forms or formulations containing conventional nontoxic pharmaceutically acceptable diluents or excipients.

The term "optionally substituted," or "optional substituents," as used herein, means that the groups in question are either unsubstituted or substituted with one or more of the substituents specified. When the groups in question are substituted with more than one substituent, the substituents may be the same or different. Moreover, when using the terms "independently," means that the groups in question may be the same or different. Certain of the herein defined terms may occur more than once in the structure, and upon such occurrence each term shall be defined independently of the other.

A "halogen" designates F, C1, Br or I. A "halogen substitution" or "halo" substitution designates replacement of one or more hydrogen atoms with F, C1, Br or I.

As used herein, the term "alkyl" refers to a saturated monovalent chain of carbon atoms, which may be optionally branched. It is understood that in embodiments that include alkyl, illustrative variations of those embodiments include lower alkyl, such as C₁ to C₉ alkyl, methyl, ethyl, propyl, 3-methylbutyl, and the like.

As used herein, the term "alkenyl" refers to an unsaturated monovalent chain of carbon atoms including at least one double bond, which may be optionally branched. It is understood that in embodiments that include alkenyl, illustrative variations of those embodiments include lower alkenyl, such as C₂ - C₆ alkenyl, and the like.

As used herein, the term "alkynyl" refers to an unsaturated monovalent chain of carbon atoms including at least one triple bond, which may be optionally branched. It is understood that in embodiments that include alkynyl, illustrative variations of those embodiments include lower alkynyl, such as C₂ - C₆ alkynyl, and the like.

As used herein, the terms "cycloalkyl" refers to a monovalent chain of carbon atoms, a portion of which forms a ring. It is understood that in embodiments that include cycloalkyl, illustrative variations of those embodiments include lower cylcoalkyl, such as C₃ - C₆ cycloalkyl, cyclopropyl, cyclobutyl, 3-methylcyclohexyl, and the like.

As used herein, the term "cycloalkenyl" refers to an unsaturated monovalent chain of carbon atoms, a portion of which forms a ring. It is understood that in embodiments that include cycloalkenyl, illustrative variations of those embodiments include lower cycloalkenyl, such as C₃ - C₆ cycloalkenyl, cyclopentenyl, cyclohexenyl, and the like.

It is understood that each of alkyl, cycloalkyl, alkenyl, and cycloalkenyl may be optionally substituted with independently selected groups such as halide, alkyl, halogenated alkyl, alkoxy, hydroxy, hydroxyalkyl, carboxylic acid and derivatives thereof, including esters, nitrile, amides, and nitrites, acyloxy, aminoalkyl and dialkylamino, acylamino, thio, and the like, and combinations thereof.

Unless indicated otherwise, percent is weight percent and ratio is weight/weight ratio. Unless indicated otherwise, weight ratio means the ratio between weight content, e.g., in an aqueous solution containing 10% solute and 90% water, the solute to water weight ratio is 1:9.

The cannabinoid-HA conjugates, according to the present invention, provide an oral delivery system for cannabinoids and synergistic compounds for the treatment of inflammation and pain with an improved pharmacokinetic/pharmacodynamic profile, compared to some other forms of oral delivery of cannabinoids.

The selection of spacer depends on many factors related to the characteristics of the spacer such as: chemical specificity and reactivity, reaction conditions, spacer chemical composition, backbone (chain) structure, solubility, spacer arm length, and spacer arm cleavability. Spacer arm length refers to the molecular span of a coupling agent or crosslinker, which defines the distance between conjugated molecules.

Because of the polymeric nature of the HA structure, there are many possible functional groups available for conjugation and derivatization. The key factor in selecting the proper site for conjugation and derivatization is the preservation of the biorecognition of the conjugated hyaluronan by the relevant transport proteins / receptors. The first preferred derivatization site is at the primary hydroxyl group (-CH₂OH) or amino group (-NH₂) of the deacetylated HA. The second preferred derivatization site is at one or more of the secondary hydroxyl groups (-OH) or the carboxylic acid moiety of HA.

Preferably, the cannabinoid is conjugated with the hyaluronic acid indirectly, by way of a spacer. In one embodiment, amino acids may be used as a spacer to conjugate the cannabinoid with hyaluronic acid. For example, the amino acid lysine may be used as a spacer to introduce an amino group to the hyaluronic acid structure, then complete the conjugation with an activated carboxyl derivative of the cannabinoid.

Alternatively, the cannabinoid may be conjugated with the hyaluronic acid directly, without the use of a spacer between the two molecules. Any suitable direct conjugation reaction may be selected, depending on the available functional groups on the hyaluronic acid and the cannabinoid. For example, where both the cannabinoid and the hyaluronic acid have carboxylic and/or hydroxyl functional groups available for conjugation, direct esterification may be used to link the hyaluronic acid and the cannabinoid molecules.

Transdermal drug delivery systems include all topically applied formulations for the purpose of delivering active pharmaceutical ingredients, or a drug, for general systemic circulation or to the skin itself. The target effect may be systemic or local, depending on the target application.

Ocular drug delivery systems include two main categories: first, the conventional ocular drug delivery systems such as topical liquid/solution eye drops, suspensions, emulsions, and ointments. Second, the non-conventional ocular drug delivery systems such as nano-based systems (such as nanoparticles, nanosuspensions, liposomes, nano-micelles and dendrimers), *in situ* gelling systems (including mucoadhesive polymers), mucoadhesive ocular dosage forms, microneedles, implants, conjunctival inserts, and contact lenses.

Different cannabinoid molecules may be attached to the same hyaluronic acid molecule through the availability of multiple conjugation sites. Due to the existence of multiple conjugation sites, hyaluronic acid can be double-functionalized with two different molecules, with different pharmacological effects, or the multiple sites can be used to increase the drug load of a single target (cargo) molecule.

The cannabinoid-hyaluronic acid conjugates may be included in oral delivery formulations which contain active ingredients, synergistic ingredients, additives, and fillers. The active ingredients are one or more cannabinoid compounds, which preferably include: tetrahydocannabinol (THC), cannabidiol (CBD), cannabinolic acid (CBNA), cannabidiolic acid (CBDA), delta-9-tetrahydrocannabinolic acid A (THCA-A), delta-8-tetrahydrocannabinolic acid (delta-8-THCA), delta-9-tetrahydrocannabiorcolic acid (THCA-C1), tetrahydrocannabivarinic acid (THCVA), cannabicycolic acid (CBLA), cannabielsoic acid A (CBEA-A), cannabielsoic acid B (CBEA-B), and derivatives thereof. More preferably, the cannabinoid compound is cannabidiolic acid.

A number of additives and/or fillers are generally used in oral delivery formulations, whether medicinal or otherwise, in order to provide the desired texture, stability, flavour, and other characteristics of the product. One or more additives or fillers are contained in the cannabinoid- hyaluronic acid conjugates oral delivery formulation to provide a desired set of characteristics to the final products, which may include: waxes, sweeteners, flavours, colours, emulsifiers, antioxidants, stabilizers, buffers, enhancers, elastomers, plasticizers, water retention agents, thickening agents, ion exchange resins, or other suitable additives and fillers for oral formulations or drinks.

As used herein, the term "conjugates" refers to the molecules obtained by combing two or compounds together either by covalent, ionic or coordination bonds. The term "conjugation" refers to the chemistry used to obtain these conjugates. A cannabinoid-HA conjugate, according to the present invention, is preferably obtained by covalent or ionic bond and may be produced by the following processes (a-e):
a) Conjugation at the carboxylic group of the D-glucuronic acid moiety may be achieved by way of amidation reaction or, as an alternative approach, by using bifunctional spacers terminated with different functional groups, then reacting the hyaluronic acid functionalized derivative with activated target molecules either directly (in the form of N-hydroxysuccinimide derivative) or indirectly with another activated spacer. Bifunctional spacers may include spacers with the general formula H₂N-spacer-FG, where FG = NH₂, COOH, or C(O)NHNH₂. The functional group (FG) can react with another activated spacer to introduce other functional groups, such as a NHS ester. Generally, in these reactions carbodiimides are used as spacers. Carbodiimides are zero-length spacers that directly conjugate amine groups to carboxylic groups, such as dicyclohexylcarbodiimide (DCC) or 1-ethyl-3-(-3-dimethylaminopropyl) cabodiimide hydrochloride (EDC). The cabodiimide molecule is not part of the linkage. Alternatively, direct amidation of carboxyl groups may be accomplished using N,N'-carbonyldiimidazole (CDI) to activate carboxylic groups under non-aqueous conditions. The activated molecule can be directly linked to amine-containing molecules.
b) Conjugation at the N-acetyl group of the N-acetyl glucosamine may be achieved by way of a two-step process. First, a deacetylation reaction to remove the acetyl group (such as reported in Zhang, W. et al. Glycoconj J (2013) 30:577-583); then in the second step the amine derivative is reacted with a carboxylic acid moiety of the target molecules via an amidation reaction to produce the conjugates. Generally, as with conjugation at the carboxylic group of the D-glucuronic acid moiety, carbodiimides are used as spacers or direct amidation of carboxyl groups may be accomplished using CDI.
c) Conjugation at the hydroxy group of the sugar backbone may be achieved by the following methods: (i) ester formation through the reaction of hyaluronic acid with acid anhydrides to form a mono or diester at the two hydroxyl groups of the saccharide moiety; or (ii) carbamate formation, which is a two-step process. First, activate the hydroxyl group either by 1,1-carbonyldiimidazole, or 1,1-carbonyl-di-(1,2,4-triazole) (CDT) at slightly elevated temperature about 30 °C. Second, react the activated hydroxyl group with an aminefunctionalized target molecule. Conjugation at the hydroxy group of the sugar backbone may also be achieved by reacting hyaluronic acid or its derivatives with an acid chloride derivative of the cannabinoid.
d) For some conjugation reactions, hyaluronic acid may be converted to an organic soluble derivative, such as tetrabutylammonium salt or cetyltrimethyl-ammonium bromide salt of hyaluronic acid to carry out the conjugation reaction in an organic solvent.
e) Different functional groups can be introduced to the hyaluronic acid structure via the carbamate method, such as amino groups, carboxylic groups, alkyne groups, azide groups, and hydrazide groups. The carbamate method can be used to incorporate a diamine spacer into hyaluronic acid, or the target molecule then conjugate this amine derivative with the N-hydroxysuccinimide (NHS) ester of the other molecule. Furthermore, these functional group would allow other possible conjugation reactions including, but not limited to carbodiimide coupling chemistry, amidation, reactive carbonyl derivatives (e.g. N,N'-Carbonyl-di-(1,2,4-triazole), Ugi condensation, ester and carbamate formation, ether and hemiacetal formation, as well as copper catalyzed and catalyzed click chemistry. The term "click chemistry" indicates the reaction of alkynes and azide groups to form biocompatible triazole adduct. The conjugates are preferably synthesized by click or carbodiimide coupling chemistry.

### Example 1: Synthesis of the HA-cannabinoid conjugates using carbodiimide coupling reagents

According to one preferred embodiment of the present invention, the cannabinoidhyaluronan conjugates, more specifically cannabinolic acid (CBNA), cannabidiolic acid (CBDA), CBD, THC or delta-9-tetrahydrocannabinolic acid A (THCA-A) and hyaluronic acid conjugates can be prepared as follows:

First, the carboxyl-containing target molecule CBNA or CBDA is activated by forming an N-hydroxysuccinimide ester by reacting the carboxylic group with NHS in the presence of DCC (N,N'-dicyclohexylcarbodiimide), as depicted below:

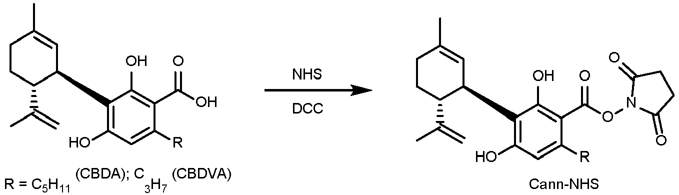

Second, the amine derivative of hyaluronic acid is prepared by deacetylation reaction of the N-acetyl group, which can be done in the presence of hydrazide sulphate over a few day at 55 °C, or by alkaline treatment using sodium hydroxide (7N), or by using enzymatic deacetylation, as depicted below:

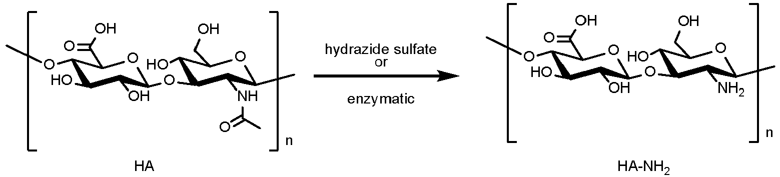

The amine derivative hyaluronic acid is then reacted with the NHS ester of the target molecule, preferably, an NHS ester of CBDA or CBDVA, to form the hyaluronic acidcannabinoid bioconjugate through the formation of an amide linkage, as shown below:

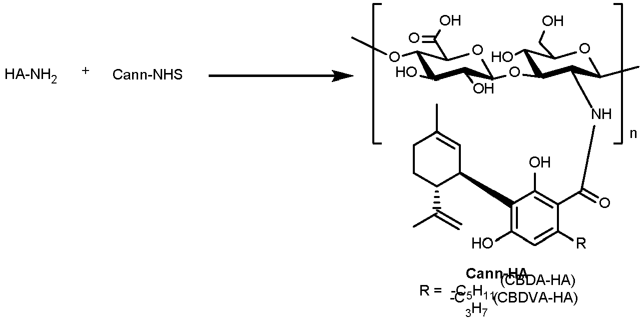

Alternatively, the activated carboxyl group of hyaluronic acid can react with a diamine spacer molecule having general formula H₂N-spacer-NH₂. Suitable diamine spacers include: ethylene diamine, diaminodipropylamine, 1,6-diaminohexane, Jeffamine EDR-148, or any diamine or PEG diamines with protected or unprotected amines, such as Poly(ethylene glycol) bis(amine), mono-N-t-boc-amido-PEG3-amine, or mono-N-t-boc-amido-PEG11-amine, or any other derivative, analogue or similar molecules.

Similar conjugates can be formed by reacting the CDT activated hyaluronic acid with aminated target molecules such as cannabinoids.

### Example 2: Synthesis of the HA-cannabinoid conjugates using Click chemistry

Starting from a commercially available azidyl HA derivative, and by using a Click chemistry protocol, HA-cannabinoid conjugates can be obtained.

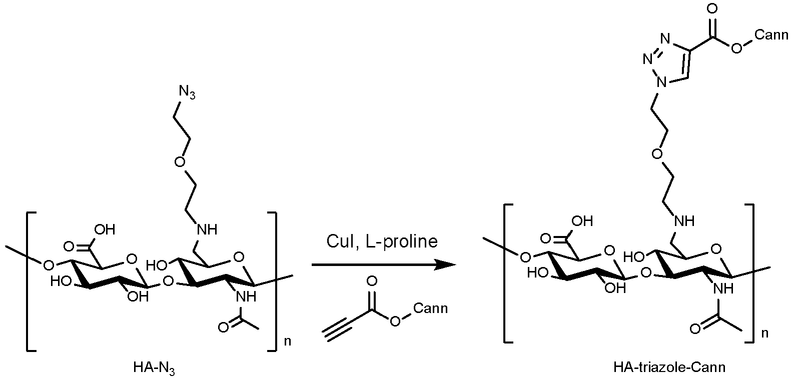

### Example 3: Synthesis of the HA-cannabinoid conjugates using reactive carbonyl reagents

Starting from deacetylated hyaluronic acid, followed by coupling of the free amino group with malonyl-Cann using carbonyldiimidazole (CDI) will furnish the desired product.

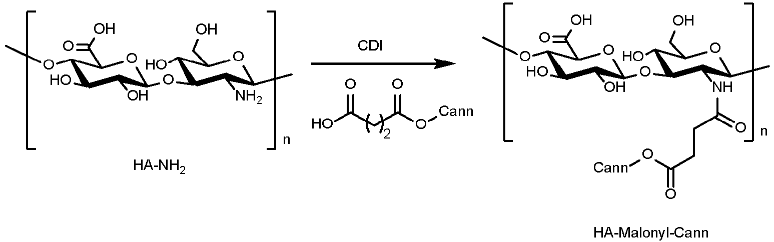

Other similar conjugates can be formed by similar and related reagents. Examples include, phosgene, trichloroacetyl chloride, 1,1'-carbonylbis(2-methylimidazole), N,N'-disuccinimidyl carbonate, 4-nitrophenylchloroformate, bis(4-nitrophenyl)carbonate, bis(pentafluorophenyl)carbonat, reactive hydrazide and dihydrazide, reactive thiols and alkenes, reactive anhydrides, sulfo-NHS, reactive alkynes and azides for click chemistry.

### Example 4: Eye drops to treat glaucoma

There is a strong evidence confirming the ability of cannabinoids to treat glaucoma by reducing the intraocular pressure (IOP). Scientific studies have confirmed that smoking marijuana plant material causes a reduction in IOP in 60% to 65% of users. Pharmacological studies have confirmed the role of cannabinoids receptors present in the eye in the IOP reduction. To control glaucoma, the level of cannabinoids needed to therapeutically control IOP cannot be achieved through smoking as significant toxic effect would be caused. The cannabinoid-hyaluronic acid conjugates provide a safer alternative to deliver therapeutic doses of cannabinoids to control IOP and treat glaucoma, in the form of eye drops or similar ocular delivery systems, without the adverse effect of smoking.

In some embodiments, the cannabinoid-hyaluronic acid conjugates can be formulated into eye drops for treating Glaucoma. The active ingredients contained in the eye drops according to the present invention is a cannabinoid-hyaluronic acid conjugate. More specifically, cannabinoid-hyaluronic acid conjugates may be formulated into eye drops in the concentration range of 0.0015 to 0.5% w/w.

The eye drop can be prepared by mixing common ingredients to adjust pH and osmolarity of the solution, preservatives, vitamins and the cannabinoid-HA conjugate at a concentration of 0.0015% w/w. The preferred pH for the eye drop is 7.4. According to one preferred embodiment of the present invention, the cannabinoid eye drops have the following composition: cannabinoid-hyaluronic acid conjugate (0.0015 - 0.5 g), gum acacia or xanthan gum (0.0 - 0.6 g), boric acid (0.5 g), sodium chloride (0.9 g), zinc sulphate (0.22 g), sodium citrate (0.2 g), benzalkonium chloride (0.01 g) or other ocular preservative, and ultra pure water, quantum satis (100 g).

### Example 5: Injection for osteoarthritis

In another embodiment, the cannabinoid-hyaluronic acid conjugates according to the present invention can be used as a knee injection to treat osteoarthritis, at 1% w/w concentration in buffered solution.

### Example 6: Topical formulation to treat skin disorders

The potential role of cannabinoids in treating skin disease has been confirmed in many publications. There is a high therapeutic potential for cannabinoids to treat skin disease such as acne, atopic dermatitis, psoriasis, skin cancer, pain, eczema and other skin disorders.

In some embodiments, the cannabinoid-hyaluronic acid conjugates can be formulated into skin cream for treating skin disorders and to facilitate skin healing. Preferably, a cannabinoid-hyaluronic acid conjugate, according to the present invention, may be contained as the active ingredient in a topical formulation in the concentration range of between 0.5 to 1% w/w.

According to one preferred embodiment of the present invention, the cannabinoids skin healing cream has the following composition: cannabinoid-hyaluronic acid conjugate (0.5 - 1.0 g), Cetyl alcohol (3.5 g), Stearic acid (4.75 g), Glycerin (3.5 g), Propylene Glycol (4.0 g), Sesame oil (2.0 g), Almond oil (3.0 g), Jojoba oil (0.5 g), argan oil (0.5 g), coconut oil (0.5 g), Polysorbitone monostereate (0.75 g), Polysorbate monoleate (1.75 g), herbal extract (0.0 - 0.65 g) and ultra pure water, quantum satis (100 g).

The cannabinoid-hyaluronic acid conjugate cream is prepared by using a phase inversion method. Using the amounts for each compound in the composition listed above, an emulsification is carried out as follows. The sesame oil, almond oil, cetyl alcohol, stearic acid, polysorbitone monostereate, polysorbate monoleate, propylene glycol and glycerin are mixed using a stirrer at 200 r.p.m. at 65-75°C on a hot plate. After complete melting and homogenous mixing, a 50 mL portion of deionized water (70 ± 2°C) is added at a rate of 30 mL/min at increased speed (300 r.p.m.). Then the temperature of the internal phase is reduced to 50°C, phase inversion takes place and the solution becomes viscous. The remaining aqueous phase containing propylene glycol is then added. The active components and, optionally, herbal extracts are then added to the base cream in propylene glycol or as an aqueous solution.

In another embodiment, other herbal ingredients may be used in the cream to provide or improve its pharmacological effects, such as synergistic antioxidant, anti-inflammatory and ultraviolet radiation protective properties. The herbal ingredients can be used in the form of an ethanolic or propylene glycol extract. The extract can be obtained by any of the extraction methods mentioned herein. The herbal extracts are preferably be obtained from the following plants: *Glycyrriza glabra, Curcuma longa, Psorolea corlifolia, Cassia tora, Areca catechu, Punica granatum, Embelica officinale, Centella asiatica, Cinnamon zeylanicum* and *Aloe vera* and the like.

In another embodiment, the skin cream may contain extracts from hemp or marijuana plants to provide the benefits of other pharmacologically active components present in hemp or marijuana plants such as terpenes, carotenoids, and other active components.

## Claims

1. A cannabinoid-hyaluronic acid conjugate, comprising a polysaccharide backbone of hyaluronic acid or a derivative thereof, having a cannabinoid attached by way of a spacer to one or more derivatization sites of the polysaccharide backbone selected from the group consisting of: a primary hydroxyl group, a secondary hydroxyl group, a carboxyl group, and an acetamido group,
wherein if the cannabinoid is CBD and the one or more derivatization sites is a primary hydroxyl group, the spacer is not O,
wherein the spacer is selected from the group consisting of: O; a linear or branched, substituted or unsubstituted alkyl chain or unsaturated carbon chain; C=O; O=C-C=O; O=C-(CH₂)nC=O, where n is any integer number; O=C-(CR₂)nC=O, where R is independently H, alkyl, halide, CN, NH2, OH, or COOH; NH-CO; NH-alkyl; substituted or unsubstituted alkyl-C=O, alkyl-NH-C=O, alkenyl-C=O, alkenyl-NH-C=O, and alkynyl-C=O; cycloalkyl, cycloalkenyl, and heterocyclic structures; azole derivatives; and triazole, optionally substituted with alkyl, carbonyl, or alkoxy moieties, and
wherein one or more of the cannabinoids is a cross-linking agent covalently cross-linking the polysaccharide backbone.

2. The cannabinoid-hyaluronic acid conjugate of claim 1, wherein the spacer is selected from the group consisting of L1, L2, L3, and L4: 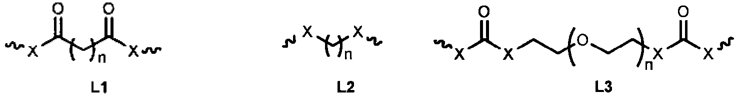 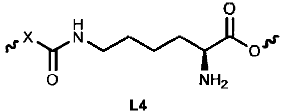 wherein, x = S, O, or NH; and, for L1 and L2, n = 1-10, and for L3, n = 1-40.

3. The cannabinoid-hyaluronic acid conjugate of claim 1, comprising one or more amide-based spacers and one or more ester-based spacers.

4. The cannabinoid-hyaluronic acid conjugate of claim 3, wherein the one or more amide-based spacers have a higher degree of covalent conjugation relative to a degree of covalent conjugation of the one or more ester-based spacers.

5. The cannabinoid-hyaluronic acid conjugate of claim 1, comprising a synergistic compound attached by way of a spacer to the one or more derivatization sites of the polysaccharide backbone.

6. The cannabinoid-hyaluronic acid conjugate of claim 5, wherein the synergistic compound is selected from the group consisting of: non-steroidal anti-inflammatory drugs (NSAIDs), functional fatty acids (FA), amino acids (AA), oligosaccharides, sugars, and vitamins.

7. The cannabinoid-hyaluronic acid conjugate of claim 6, wherein the synergistic compound is selected from the group consisting of: diclofenac sodium, oleic acid, stearic acid, linolenic acid, palmitic acid, caproic acid, arachidonic acid, anandamide, phosphatidylethanolamine, leucine, isoleucine, lysine, valine, glucosamine, and vitamin C.

8. The cannabinoid-hyaluronic acid conjugate of claim 1, wherein the polysaccharide backbone comprises a derivative of hyaluronic acid selected from the group consisting of: chondroitin sulfate, chitosan, polyacrylic acid carboxypolymethylene, carboxymethylcellulose, and hyaluronate.

9. The cannabinoid-hyaluronic acid conjugate of claim 1, wherein the cannabinoid is selected from the group consisting of: delta-9-tetrahydrocannabinol (THC), cannabidiol (CBD), cannabinol (CBN), cannabinolic acid (CBNA), cannabigerol (CBG), cannabigerovarin (CBGV), cannabichromene (CBC), cannabicyclol (CBL), canabivarol (CBV), tetrahydrocannabivarin (THCV), cannabidivarin (CBDV), cannabichromevarin (CBCV), cannabigerol monoethyl ether (CBGM), cannabigerolic acid monoethyl ether (CBGAM) cannabidiolic acid (CBDA), cannabigerovarinic acid (CBGVA), cannabichromenic acid (CBCA), cannabidiol monomethylether (CBDM), cannabidiol-C₄ (CBD-C₄), cannabidivarinic acid (CBDVA), cannabidiorcol (CBD-C₁), delta-9-tetrahydrocannabinolic acid A (THCA-A), delta-9-tetrahydrocannabinolic acid B (THCA-B), delta-9-tetrahydrocannabinolic acid-C₄ (THCA-C₄), delta-8-tetrahydrocannabinolic acid (delta-8-THCA), delta-8-tetrahydrocannabinol (delta-8-THC), delta-9-tetrahydrocannabinol-C₄ (THC-C₄), delta-9-tetrahydrocannabiorcolic acid (THCA-C₁), delta-9-tetrahydrocannabiorcol-C₁ (THC-C₁), tetrahydrocannabivarinic acid (THCVA), cannabicycolic acid (CBLA), cannbicyclol (CBL), cannabicyclovarin (CBLV), cannabielsoic acid A (CBEA-A), cannabielsoic acid B (CBEA-B), cannabielsoin (CBE), cannabivarin, cannabinol-C₄ (CBN-C₄), cannabinol methylether (CBNM), cannabiorcol (CBN-C₁), cannabinol-C₂ (CBN-C₂), cannabinodiol (CBND), cannabinodivarin (CBVD), cannabitriol (CBT), cannabitriolvarin (CBTV), dehydrocannabifuran (DCBF), cannabifuran, cannabicitran (CBT), cannabiripsol (CBR), '11-hydroxytetrahydrocannabinol' (11-OH-THC), '11-nor-9-carboxy-tetrahydrocannabinol' (THC-COOH), and derivatives, analogues, mixtures, and combinations thereof.

10. The cannabinoid-hyaluronic acid conjugate of claim 1, wherein the degree of crosslinking is between 5% and 50% of the available cross-linking groups.

11. The cannabinoid-hyaluronic acid conjugate of claim 10, wherein the degree of crosslinking is between 10% and 40% of the available cross-linking groups.

12. The cannabinoid-hyaluronic acid conjugate of claim 11, wherein the degree of crosslinking is between 20% and 40% of the available cross-linking groups.

13. A compound having a formula of Compound I: 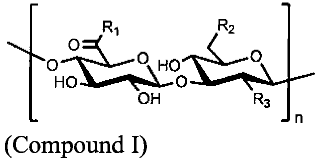 wherein: n = a repeating unit of the compound;
R₁ = -ONa, -OH, or a cannabinoid attached by way of a spacer;
R₂ = -OH or a cannabinoid attached by way of a spacer, wherein if the cannabinoid is CBD the spacer is not O; and
R₃ = -NHCOCH₃ or a cannabinoid attached by way of a spacer;
and wherein at least one of R₁, R₂, or R₃ is a cannabinoid attached by way of a spacer, independently in one or more of the repeating units; and
wherein one or more of the cannabinoids is a cross-linking agent covalently cross-linking the polysaccharide backbone.

14. The compound of claim 13, wherein the spacer is selected from the group consisting of: O; a linear or branched, substituted or unsubstituted alkyl chain or unsaturated carbon chain; C=O; O=C-C=O; O=C-(CH₂)nC=O, where n is any integer number; O=C-(CR₂)nC=O, where R is independently H, alkyl, halide, CN, NH2, OH, or COOH; NH-CO; NH-alkyl; substituted or unsubstituted alkyl-C=O, alkyl-NH-C=O, alkenyl-C=O, alkenyl-NH-C=O, and alkynyl-C=O; cycloalkyl, cycloalkenyl, and heterocyclic structures; azole derivatives; and triazole, optionally substituted with alkyl, carbonyl, or alkoxy moieties.

15. The compound of claim 14, wherein the spacer is selected from the group consisting of L1, L2, L3, and L4: 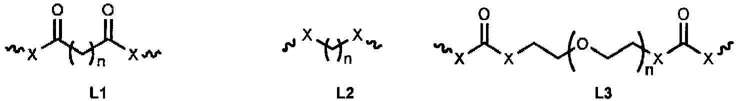 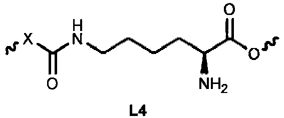 wherein, x = S, O, or NH; and, for L1 and L2, n = 1-10, and for L3, n = 1-40.

16. The compound of claim 15, wherein a second cannabinoid is attached by way of a spacer to one or R₁, R₂, or R₃, independently in one or more of the repeating units.

17. The compound of claim 13, wherein in one or more of the repeating units:
R₁ = -ONa or -OH;
R₂ = -OH; and
R₃=

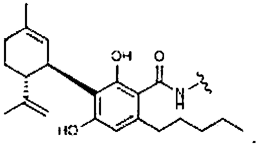

18. The compound of claim 13, wherein in one or more of the repeating units:
R₁ = -ONa or -OH;
R₂= 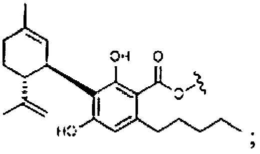 and
R₃ = -NHCOCH₃.

19. The compound of claim 13, wherein in one or more of the repeating units:
R₁= 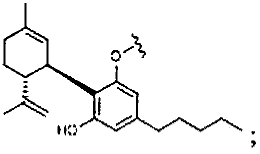
R₂ = -OH; and
R₃ = -NHCOCH₃.

20. The compound of claim 13, wherein in one or more of the repeating units:
R₁ = -ONa or -OH; and
R₂= 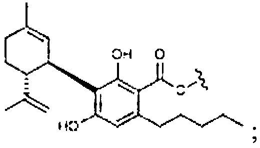 and
R₃ =

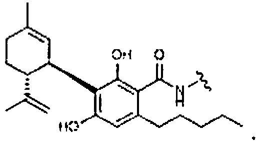

21. The compound of claim 13, wherein in one or more of the repeating units:
R₁ = -ONa or -OH;
R₂ = 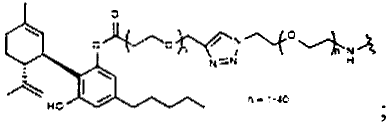 and
R₃ = -NHCOCH₃.

22. The compound of claim 13, wherein in one or more of the repeating units:
R₁ = -ONa or -OH;
R₂ = -OH; and
R₃ =

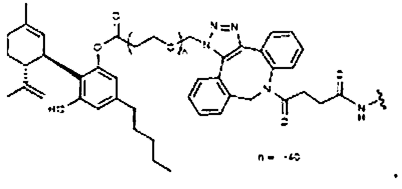

23. The compound of claim 13, wherein in one or more of the repeating units:
R₁ = -ONa or -OH;
R₂ = 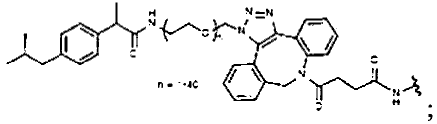 and
R₃=

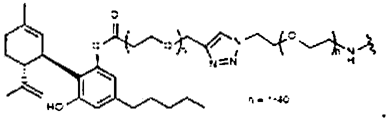

24. The compound of claim 13, wherein in one or more of the repeating units:
R₁ = -ONa or -OH;
R₂ = 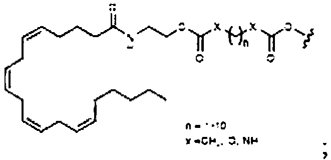 and
R₃=

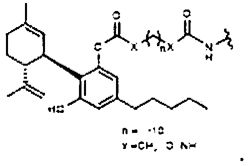

25. The compound of claim 13, wherein in one or more of the repeating units:
R₁ = -ONa or -OH;
R₂ = 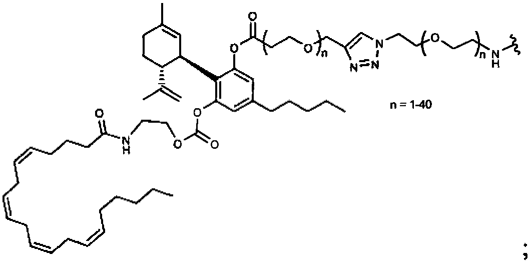 and
R₃ = -NHCOCH₃.

26. The compound of claim 13, wherein in one or more of the repeating units:
R₁ = -ONa or -OH;
R₂ **=** -OH; and
R₃=

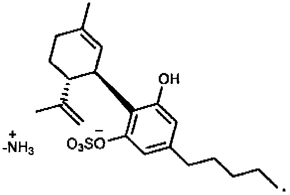

27. The compound of claim 13, wherein in one or more of the repeating units:
R₁ = -ONa or -OH;
R₂ = -OH; and
R₃=

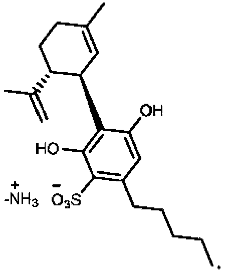

28. The compound of claim 13, wherein more than one of R₁, R₂, or R₃ is a cannabinoid attached by way of a spacer, independently in one or more of the repeating units.

29. A compound having a formula of Compound II: 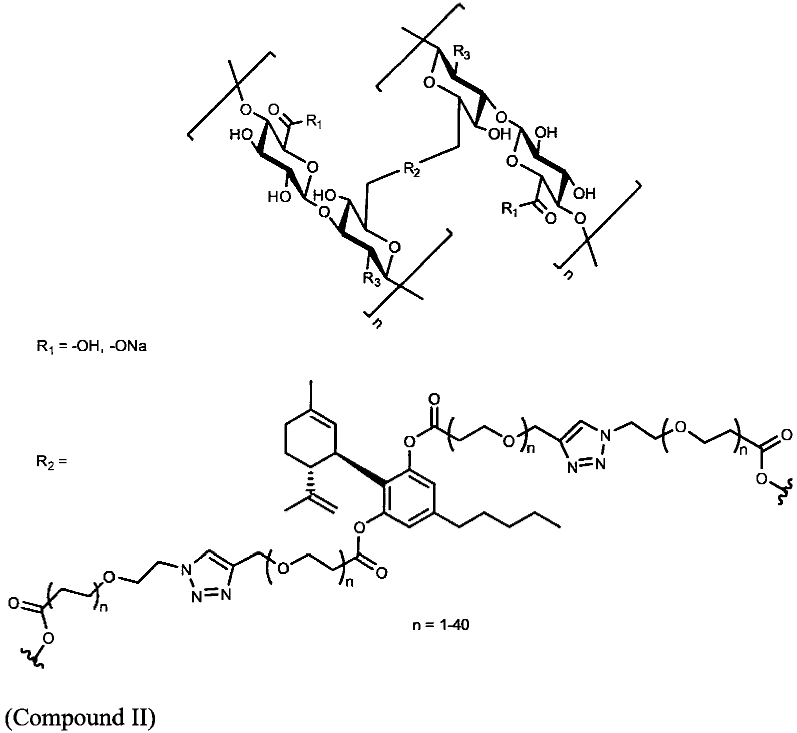
wherein: n = a repeating unit of the compound; and
R₃ = -NHCOCH₃ or a cannabinoid attached by way of a spacer;
and wherein R₃ is a cannabinoid attached by way of a spacer, independently in one or more of the repeating units.

30. The compound of claim 29, wherein R₃ is

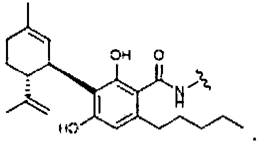

31. The compound of claim 29, wherein R₃ is

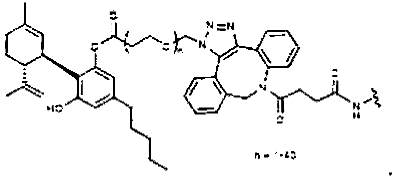

32. The compound of claim 29, wherein R₃ is

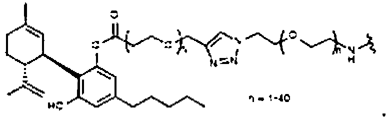

33. The compound of claim 29, wherein R₃ is

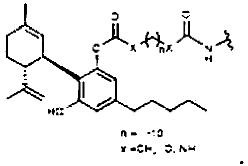

34. The compound of claim 29, wherein R₃ is

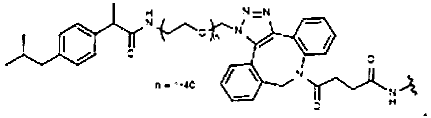

35. The compound of claim 29, wherein R₃ is

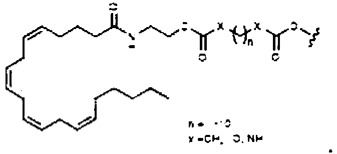

36. The compound of claim 29, wherein R₃ is

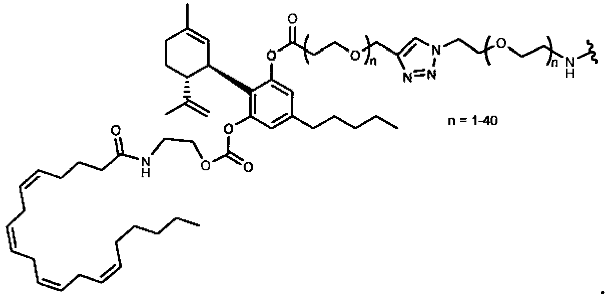

37. A pharmaceutical formulation in the form of eye drops for the treatment of glaucoma, comprising a cannabinoid-hyaluronic acid conjugate according to claim 1.

38. The pharmaceutical formulation of claim 37, comprising: : the cannabinoid-hyaluronic acid conjugate in an amount between 0.0015% w/w and 0.5% w/w; gum acacia or xantham gum in an amount less than 0.6% w/w; boric acid in an amount of 0.5% w/w; sodium chloride in an amount of 0.9% w/w; sinc sulphate in an amount of 0.22% w/w; sodium citrate in an amount of 0.2% w/w; benzalkonium chloride in an amount of 0.01% w/w.

39. A pharmaceutical formulation in the form of an injection for the treatment of osteoarthritis, comprising a cannabinoid-hyaluronic acid conjugate according to claim 1.

40. A topical formulation for the treatment of skin disorders, comprising a cannabinoid-hyaluronic acid conjugate according to claim 1.

41. The topical formulation of claim 40, comprising: the cannabinoid-hyaluronic acid conjugate in an amount between 0.5% and 1% w/w, cetyl alcohol in an amount of 3.5% w/w, stearic acid in an amount of 4.75% w/w, glycerin in an amount of 3.5% w/w, propylene glycol in an amount of 4.0% w/w, sesame oil in an amount of 2.0% w/w, almond oil in an amount of 3.0%w/w, jojoba oil in an amount of 0.5% w/w, argan oil in an amount of 0.5% w/w, coconut oil in an amount of 0.5% w/w, polysorbitone monostereate in an amount of 0.75% w/w, polysorbate monoleate in an amount of 1.75% w/w, and a herbal extract in an amount less than 0.65%

42. The topical formulation of claim 41, wherein the herbal extract is an extract from a plant selected from the group consisting of: *Glycyrriza glabra, Curcuma longa, Psorolea corlifolia, Cassia tora, Areca catechu, Punica granatum, Embelica officinale, Centella asiatica, Cinnamon zeylanicum* and *Aloe vera.*

43. The topical formulation of claim 41, wherein the herbal extract is an extract from hemp or marijuana plants.

## Patentansprüche

1. Cannabinoid-Hyaluronsäure-Konjugat, umfassend ein Polysaccharidgerüst aus Hyaluronsäure oder einem Derivat davon mit einem über einen aus der aus einer primären Hydroxylgruppe, einer sekundären Hydroxylgruppe, einer Carboxylgruppe und einer Acetamidogruppe bestehenden Gruppe ausgewählten Spacer an eine oder mehrere Derivatisierungsstellen des Polysaccharidgerüsts gebundenen Cannabinoid,
wobei, wenn das Cannabinoid CBD ist und die eine oder die mehreren Derivatisierungsstellen eine primäre Hydroxylgruppe sind, der Spacer nicht O ist,
wobei der Spacer aus der aus den Folgenden bestehenden Gruppe ausgewählt ist: O; eine geradkettige oder verzweigte, substituierte oder unsubstituierte Alkylkette oder ungesättigte Kohlenstoffkette; C=O; O=C-C=O; O=C-(CH₂)nC=O, wobei n für eine beliebige ganze Zahl steht; O=C-(CR₂)nC=O, wobei R unabhängig für H, Alkyl, Halogenid, CN, NH₂, OH oder COOH steht; NH-CO; NH-Alkyl; substituiertem oder unsubstituiertem Alkyl-C=O, Alkyl-NH-C=O, Alkenyl-C=O, Alkenyl-NH-C=O, und Alkinyl-C=O; Cycloalkyl, Cycloalkenyl und heterocyclischen Strukturen; Azolderivaten und Triazol, gegebenenfalls substituiert durch Alkyl-, Carbonyl- oder Alkoxygruppierungen, und
wobei eines oder mehrere der Cannabinoide ein Vernetzungsmittel ist, das das Polysaccharidgerüst kovalent vernetzt.

2. Cannabinoid-Hyaluronsäure-Konjugat nach Anspruch 1, wobei der Spacer aus der aus L1, L2, L3 und L4 bestehenden Gruppe ausgewählt ist: 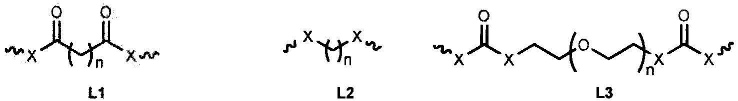 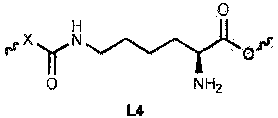 wobei x = S, O oder NH und, für L1 und L2, n = 1-10 und, für L3, n = 1-40.

3. Cannabinoid-Hyaluronsäure-Konjugat nach Anspruch 1, umfassend einen oder mehrere amidbasierte Spacer und einen oder mehrere esterbasierte Spacer.

4. Cannabinoid-Hyaluronsäure-Konjugat nach Anspruch 3, wobei der eine oder die mehreren amidbasierten Spacer einen höheren Grad an kovalenter Konjugation im Vergleich zu einem Grad an kovalenter Konjugation des einen oder der mehreren esterbasierten Spacer aufweisen.

5. Cannabinoid-Hyaluronsäure-Konjugat nach Anspruch 1, umfassend eine synergistische Verbindung, die über einen Spacer an die eine oder die mehreren Derivatisierungsstellen des Polysaccharidgerüsts gebunden ist.

6. Cannabinoid-Hyaluronsäure-Konjugat nach Anspruch 5, wobei die synergistische Verbindung aus der aus nichtsteroidalen entzündungshemmenden Arzneimitteln (NSAIDs), funktionellen Fettsäuren (FA), Aminosäuren (AA), Oligosacchariden, Zuckern und Vitaminen bestehenden Gruppe ausgewählt ist.

7. Cannabinoid-Hyaluronsäure-Konjugat nach Anspruch 6, wobei die synergistische Verbindung aus der aus Diclofenac-Natrium, Ölsäure, Stearinsäure, Linolensäure, Palmitinsäure, Capronsäure, Arachidonsäure, Anandamid, Phosphatidylethanolamin, Leucin, Isoleucin, Lysin, Valin, Glucosamin und Vitamin C bestehenden Gruppe ausgewählt ist.

8. Cannabinoid-Hyaluronsäure-Konjugat nach Anspruch 1, wobei das Polysaccharidgerüst ein aus der aus Chondroitinsulfat, Chitosan, Polyacrylsäurecarboxypolymethylen, Carboxymethylcellulose und Hyaluronat bestehenden Gruppe ausgewähltes Derivat von Hyaluronsäure umfasst.

9. Cannabinoid-Hyaluronsäure-Konjugat nach Anspruch 1, wobei das Cannabinoid aus der aus delta-9-Tetrahydrocannabinol (THC), Cannabidiol (CBD), Cannabinol (CBN), Cannabinolsäure (CBNA), Cannabigerol (CBG), Cannabigerovarin (CBGV), Cannabichromen (CBC), Cannabicyclol (CBL), Canabivarol (CBV), Tetrahydrocannabivarin (THCV), Cannabidivarin (CBDV), Cannabichromevarin (CBCV), Cannabigerolmonoethylether (CBGM), Cannabigerolsäuremonoethylether (CBGAM), Cannabidiolsäure (CBDA), Cannabigerovarinicsäure (CBGVA), Cannabichromensäure (CBCA), Cannabidiolmonomethylether (CBDM), Cannabidiol-C₄ (CBD-C₄), Cannabidivarinicsäure (CBDVA), Cannabidiorcol (CBD-C₁), delta-9-Tetrahydrocannabinolsäure A (THCA-A), delta-9-Tetrahydrocannabinolsäure B (THCA-B), delta-9-Tetrahydrocannabinolsäure-C₄ (THCA-C₄), delta-8-Tetrahydrocannabinolsäure (delta-8-THCA), delta-8-Tetrahydrocannabinol (delta-8-THC), delta-9-Tetrahydrocannabinol-C₄ (THC-C₄), delta-9-Tetrahydrocannabiorcolsäure (THCA-C₁), delta-9-Tetrahydrocannabiorcol-C₁ (THC-C₁), Tetrahydrocannabivarinsäure (THCVA), Cannabicycolsäure (CBLA), Cannbicyclol (CBL), Cannabicyclovarin (CBLV), Cannabielsoinsäure A (CBEA-A), Cannabielsoinsäure B (CBEA-B), Cannabielsoin (CBE), Cannabivarin, Cannabinol-C₄ (CBN-C₄), Cannabinolmethylether (CBNM), Cannabiorcol (CBN-C₁), Cannabinol-C₂ (CBN-C₂), Cannabinodiol (CBND), Cannabinodivarin (CBVD), Cannabitriol (CBT), Cannabitriolvarin (CBTV), Dehydrocannabifuran (DCBF), Cannabifuran, Cannabicitran (CBT), Cannabiripsol (CBR), '11-Hydroxytetrahydrocannabinol' (11-OH-THC), '11-nor-9-Carboxytetrahydrocannabinol' (THC-COOH) und Derivaten, Analoga, Mischungen und Kombinationen davon bestehenden Gruppe ausgewählt ist.

10. Cannabinoid-Hyaluronsäure-Konjugat nach Anspruch 1, wobei der Vernetzungsgrad zwischen 5 % und 50 % der verfügbaren Vernetzungsgruppen beträgt.

11. Cannabinoid-Hyaluronsäure-Konjugat nach Anspruch 10, wobei der Vernetzungsgrad zwischen 10 % und 40 % der verfügbaren Vernetzungsgruppen beträgt.

12. Cannabinoid-Hyaluronsäure-Konjugat nach Anspruch 11, wobei der Vernetzungsgrad zwischen 20 % und 40 % der verfügbaren Vernetzungsgruppen beträgt.

13. Verbindung mit einer Formel von Verbindung I: 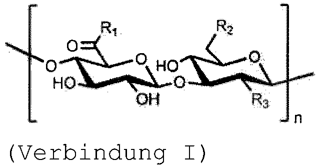
wobei: n = eine Wiederholungseinheit der Verbindung,
R₁ = -ONa, -OH oder ein Cannabinoid, das über einen Spacer gebunden ist,
R₂ = -OH oder ein Cannabinoid, das über einen Spacer gebunden ist, wobei, wenn das Cannabinoid CBD ist, der Spacer nicht O ist, und
R₃ = -NHCOCH₃ oder ein Cannabinoid, das über einen Spacer gebunden ist,
und wobei mindestens eines von R₁, R₂ oder R₃ für ein Cannabinoid steht, das in einer oder mehreren der Wiederholungseinheiten unabhängig über einen Spacer gebunden ist, und
wobei eines oder mehrere der Cannabinoide ein Vernetzungsmittel ist, das das Polysaccharidgerüst kovalent vernetzt.

14. Verbindung nach Anspruch 13, wobei der Spacer aus der aus den Folgenden bestehenden Gruppe ausgewählt ist: O; einer geradkettigen oder verzweigten, substituierten oder unsubstituierten Alkylkette oder ungesättigten Kohlenstoffkette; C=O; O=C-C=O; O=C-(CH₂)nC=O, wobei n für eine beliebige ganze Zahl steht; O=C-(CR₂)nC=O, wobei R unabhängig für H, Alkyl, Halogenid, CN, NH₂, OH oder COOH steht; NH-CO; NH-Alkyl; substituiertem oder unsubstituiertem Alkyl-C=O, Alkyl-NH-C=O, Alkenyl-C=O, Alkenyl-NH-C=O, und Alkinyl-C=O; Cycloalkyl, Cycloalkenyl und heterocyclischen Strukturen; Azolderivaten und Triazol, gegebenenfalls substituiert durch Alkyl-, Carbonyl- oder Alkoxygruppierungen.

15. Verbindung nach Anspruch 14, wobei der Spacer aus der aus L1, L2, L3 und L4 bestehenden Gruppe ausgewählt ist: 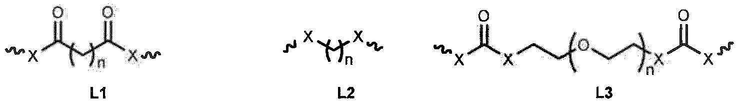 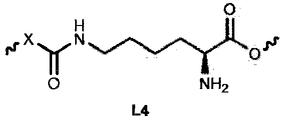 wobei x = S, O oder NH und, für L1 und L2, n = 1-10 und, für L3, n = 1-40.

16. Verbindung nach Anspruch 15, wobei in einer oder mehreren der Wiederholungseinheiten unabhängig ein zweites Cannabinoid über einen Spacer an einen oder R₁, R₂ oder R₃ gebunden ist.

17. Verbindung nach Anspruch 13, wobei in einer oder mehreren der Wiederholungseinheiten:
R₁ = -ONa oder -OH,
R₂ = -OH und
R₃=

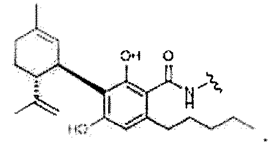

18. Verbindung nach Anspruch 13, wobei in einer oder mehreren der Wiederholungseinheiten:
R₁ = -ONa oder -OH,
R₂= 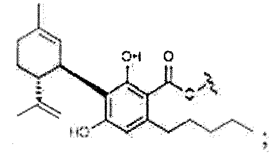 und
R₃ = -NHCOCH₃.

19. Verbindung nach Anspruch 13, wobei in einer oder mehreren der Wiederholungseinheiten:
R₁= 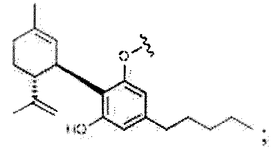
R₂ = -OH und
R₃ = -NHCOCH₃.

20. Verbindung nach Anspruch 13, wobei in einer oder mehreren der Wiederholungseinheiten:
R₁ = -ONa oder -OH und
R₂= 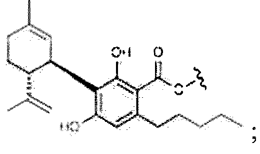 und
R₃=

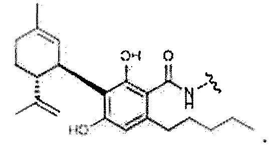

21. Verbindung nach Anspruch 13, wobei in einer oder mehreren der Wiederholungseinheiten:
R₁ = -ONa oder -OH,
R₂= 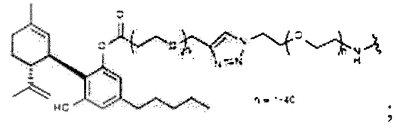 und
R₃ = -NHCOCH₃ .

22. Verbindung nach Anspruch 13, wobei in einer oder mehreren der Wiederholungseinheiten:
R₁ = -ONa oder -OH,
R₂ = -OH und
R₃=

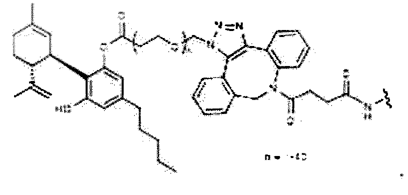

23. Verbindung nach Anspruch 13, wobei in einer oder mehreren der Wiederholungseinheiten:
R₁ = -ONa oder -OH,
R₂= 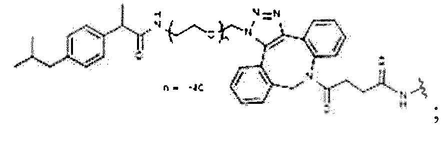 und
R₃=

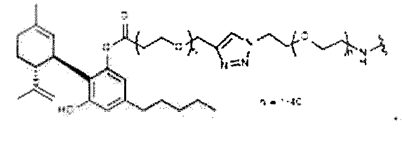

24. Verbindung nach Anspruch 13, wobei in einer oder mehreren der Wiederholungseinheiten:
R₁ = -ONa oder -OH,
R₂= 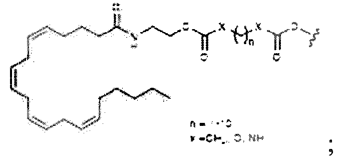 und
R₃=

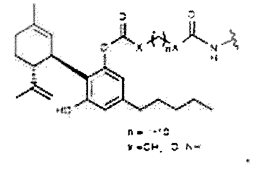

25. Verbindung nach Anspruch 13, wobei in einer oder mehreren der Wiederholungseinheiten:
R₁ = -ONa oder -OH,
R₂= 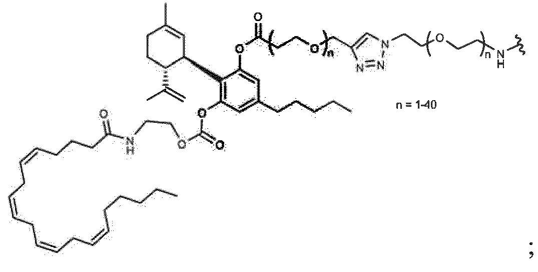 und
R₃ = -NHCOCH₃.

26. Verbindung nach Anspruch 13, wobei in einer oder mehreren der Wiederholungseinheiten:
R₁ = -ONa oder -OH,
R₂ = -OH und
R₃=

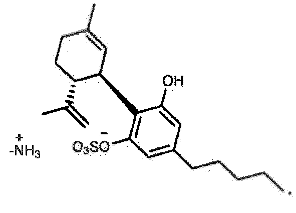

27. Verbindung nach Anspruch 13, wobei in einer oder mehreren der Wiederholungseinheiten:
R₁ = -ONa oder -OH,
R₂ = -OH und
R₃=

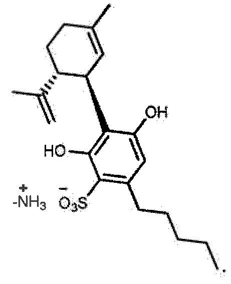

28. Verbindung nach Anspruch 13, wobei mehr als eines von R₁, R₂ oder R₃ für ein Cannabinoid steht, das in einer oder mehreren der Wiederholungseinheiten unabhängig über einen Spacer gebunden ist.

29. Verbindung mit einer Formel von Verbindung II: 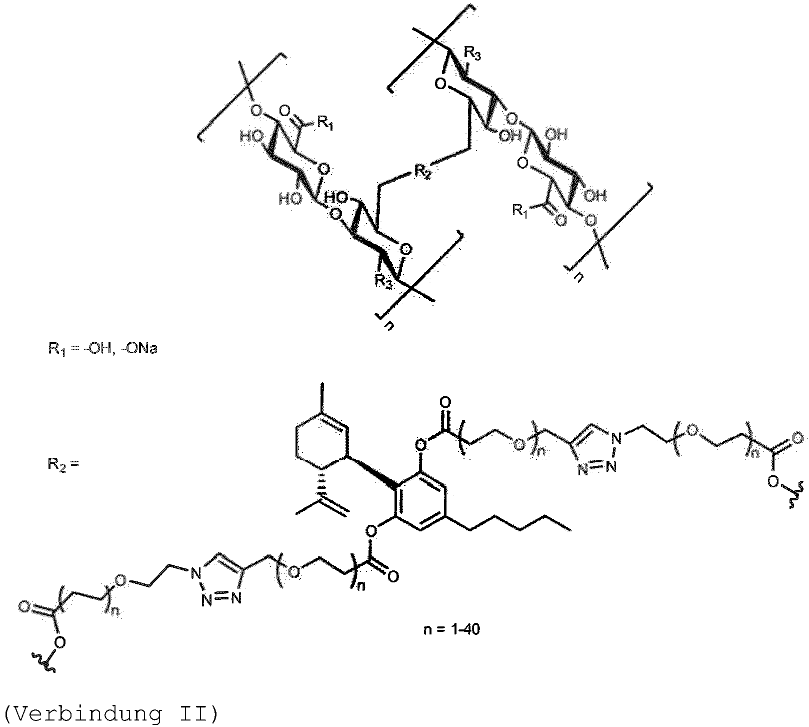
wobei: n = eine Wiederholungseinheit der Verbindung und R₃ = -NHCOCH₃ oder ein Cannabinoid, das über einen Spacer gebunden ist,
und wobei R₃ für ein Cannabinoid steht, das in einer oder mehreren der Wiederholungseinheiten unabhängig über einen Spacer gebunden ist.

30. Verbindung nach Anspruch 29, wobei R₃ für Folgendes steht:

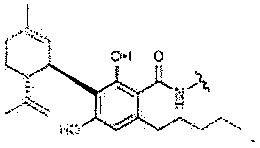

31. Verbindung nach Anspruch 29, wobei R₃ für Folgendes steht:

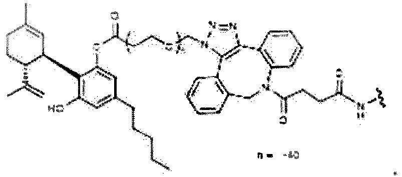

32. Verbindung nach Anspruch 29, wobei R₃ für Folgendes steht:

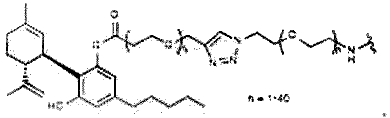

33. Verbindung nach Anspruch 29, wobei R₃ für Folgendes steht:

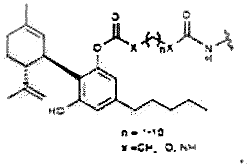

34. Verbindung nach Anspruch 29, wobei R₃ für Folgendes steht:

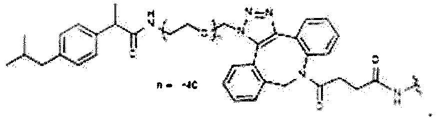

35. Verbindung nach Anspruch 29, wobei R₃ für Folgendes steht:

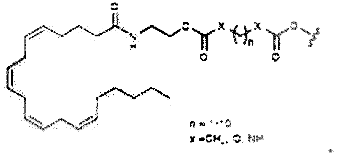

36. Verbindung nach Anspruch 29, wobei R₃ für Folgendes steht:

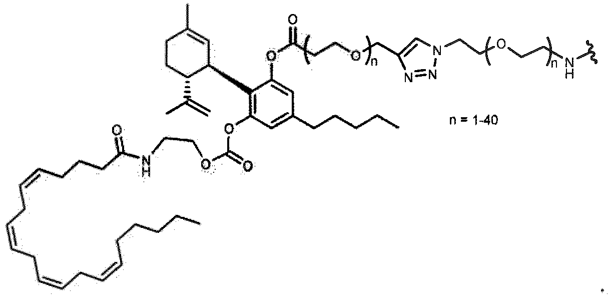

37. Pharmazeutische Formulierung in Form von Augentropfen zur Behandlung von Glaukom, umfassend ein Cannabinoid-Hyaluronsäure-Konjugat nach Anspruch 1.

38. Pharmazeutische Formulierung nach Anspruch 37, umfassend: Das Cannabinoid-Hyaluronsäure-Konjugat in einer Menge zwischen 0,0015 Gew.-% und 0,5 Gew.-%; Gummi arabicum oder Gummi Xanthan in einer Menge von weniger als 0,6 Gew.-%; Borsäure in einer Menge von 0,5 Gew.-%; Natriumchlorid in einer Menge von 0,9 Gew.-%; Zinksulfat in einer Menge von 0,22 Gew.-%, Natriumcitrat in einer Menge von 0,2 Gew.-%, Benzalkoniumchlorid in einer Menge von 0,01 Gew.-%.

39. Pharmazeutische Formulierung in Form eines Injektionspräparates zur Behandlung von Osteoarthritis, umfassend ein Cannabinoid-Hyaluronsäure-Konjugat nach Anspruch 1.

40. Topische Formulierung zur Behandlung von Hauterkrankungen, umfassend ein Cannabinoid-Hyaluronsäure-Konjugat nach Anspruch 1.

41. Topische Formulierung nach Anspruch 40, umfassend: Das Cannabinoid-Hyaluronsäure-Konjugat in einer Menge zwischen 0,5 Gew.-% und 1 Gew.-%, Cetylalkohol in einer Menge von 3,5 Gew.-%, Stearinsäure in einer Menge von 4,75 Gew.-%, Glycerin in einer Menge von 3,5 Gew.-%, Propylenglykol in einer Menge von 4,0 Gew.-%, Sesamöl in einer Menge von 2,0 Gew.-%, Mandelöl in einer Menge von 3,0 Gew.-%, Jojobaöl in einer Menge von 0,5 Gew.-%, Arganöl in einer Menge von 0,5 Gew.-%, Kokosöl in einer Menge von 0,5 Gew.-%, Polysorbitonmonostereat in einer Menge von 0,75 Gew.-%, Polysorbatmonoleat in einer Menge von 1,75 Gew.-% und einen Kräuterextrakt in einer Menge von weniger als 0,65 Gew.-%.

42. Topische Formulierung nach Anspruch 41, wobei der Kräuterextrakt ein Extrakt aus einer aus der aus den folgenden bestehenden Gruppe ausgewählten Pflanze ist: Glycyrriza glabra, Curcuma longa, Psorolea corlifolia, Cassia tora, Areca catechu, Punica granatum, Embelica officinale, Centella asiatica, Cinnamon zeylanicum und Aloe vera.

43. Topische Formulierung nach Anspruch 41, wobei der Kräuterextrakt ein Extrakt aus Hanf- oder Marihuanapflanzen ist.

## Revendications

1. Conjugué cannabinoïde-acide hyaluronique, comprenant un squelette polysaccharidique d'acide hyaluronique ou d'un dérivé de celui-ci, comportant un cannabinoïde lié par l'intermédiaire d'un espaceur à un ou plusieurs sites de dérivatisation du squelette polysaccharidique choisis dans le groupe constitué par : un groupe hydroxyle primaire, un groupe hydroxyle secondaire, un groupe carboxyle et un groupe acétamido, dans lequel, si le cannabinoïde est le CBD et le ou les sites de dérivatisation sont un groupe hydroxyle primaire, l'espaceur n'est pas O,
dans lequel l'espaceur est choisi dans le groupe constitué par : O ; une chaîne alkyle ou une chaîne carbonée insaturée, linéaire ou ramifiée, substituée ou non ; C=O ; O=C-C=O ; O=C-(CH₂)nC=O où n est un nombre entier quelconque ; O=C- (CR₂)nC=O où R représente indépendamment H, un groupe alkyle, halogénure, CN, NH2, OH ou COOH ; NH-CO ; NH-alkyle ; alkyl-C=O substitués ou non substitués, alkyl-NH-C=O, alcényl-C=O, alcényl-NH-C=O, et alcynyl-C=O; des structures cycloalkyle, cycloalcényle et hétérocycliques ; des dérivés d'azole ; et triazole, facultativement substitué par des groupes alkyle, carbonyle ou alcoxy, et
dans lequel un ou plusieurs des cannabinoïdes sont un agent de réticulation réticulant de manière covalente le squelette polysaccharidique.

2. Conjugué cannabinoïde-acide hyaluronique selon la revendication 1, dans lequel l'espaceur est choisi dans le groupe constitué par L1, L2, L3 et L4 : 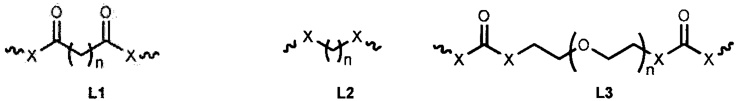 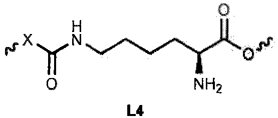 dans lequel, x = S, O ou NH ; et, pour L1 et L2, n = 1 à 10, et pour L3, n = 1 à 40.

3. Conjugué cannabinoïde-acide hyaluronique selon la revendication 1, comprenant un ou plusieurs espaceurs à base d'amide et un ou plusieurs espaceurs à base d'ester.

4. Conjugué cannabinoïde-acide hyaluronique selon la revendication 3, dans lequel le ou les espaceurs à base d'amide ont un degré de conjugaison covalente supérieur par rapport au degré de conjugaison covalente du ou des espaceurs à base d'ester.

5. Conjugué cannabinoïde-acide hyaluronique selon la revendication 1, comprenant un composé synergique lié par l'intermédiaire d'un espaceur au ou aux sites de dérivatisation du squelette polysaccharidique.

6. Conjugué cannabinoïde-acide hyaluronique selon la revendication 5, dans lequel le composé synergique est choisi dans le groupe constitué par : des médicaments anti-inflammatoires non stéroïdiens (AINS), des acides gras fonctionnels (FA), des acides aminés (AA), des oligosaccharides, des sucres et des vitamines.

7. Conjugué cannabinoïde-acide hyaluronique selon la revendication 6, dans lequel le composé synergique est choisi dans le groupe constitué par : diclofénac sodium, acide oléique, acide stéarique, acide linolénique, acide palmitique, caproïque, acide arachidonique, anandamide, phosphatidyléthanolamine, leucine, isoleucine, lysine, valine, glucosamine et vitamine C.

8. Conjugué cannabinoïde-acide hyaluronique selon la revendication 1, dans lequel le squelette polysaccharidique comprend un dérivé d'acide hyaluronique choisi dans le groupe constitué par : sulfate de chondroïtine, chitosane, acide polyacrylique carboxypolyméthylène, carboxyméthylcellulose et hyaluronate.

9. Conjugué cannabinoïde-acide hyaluronique selon la revendication 1, dans lequel le cannabinoïde est choisi dans le groupe constitué par : delta-9-tétrahydrocannabinol (THC), cannabidiol (CBD), cannabinol (CBN), acide cannabinolique (CBNA), cannabigérol (CBG), cannabigérovarine (CBGV), cannabichromène (CBC), cannabicyclol (CBL), canabivarol (CBV), tétrahydrocannabivarine (THCV), cannabidivarine (CBDV), cannabichromévarine (CBCV), éther monoéthylique de cannabigérol (CBGM), éther monoéthylique d'acide cannabigérolique (CBGAM), acide cannabidiolique (CBDA), acide cannabigérovarinique (CBGVA), acide cannabichroménique (CBCA), éther monométhylique de cannabidiol (CBDM), cannabidiol-C₄ (CBD-C₄) , acide cannabidivarinique (CBDVA), cannabidiorcol (CBD-C1), acide delta-9-tétrahydrocannabinolique A (THCA-A), acide delta-9-tétrahydrocannabinolique B (THCA-B), acide delta-9-tétrahydrocannabinolique-C₄ (THCA-C₄), acide delta-8-tétrahydrocannabinolique (delta-8-THCA), delta-8-tétrahydrocannabinol (delta-8-THC), delta-9-tétrahydrocannabinol-C₄ (THC-C₄) , acide delta-9-tétrahydrocannabiorcolique (THCA-C₁), delta-9-tétrahydrocannabiorcol-C₁ (THC-C₁), acide tétrahydrocannabivarinique (THCVA), acide cannabicycolique (CBLA), canbicyclol (CBL), cannabicyclovarine (CBLV), acide cannabielsoïque A (CBEA-A), acide cannabielsoïque B (CBEA-B), cannabielsoïne (CBE), cannabivarine, cannabinol-C₄ (CBN-C₄), éther méthylique de cannabinol (CBNM), cannabiorcol (CBN-C1), cannabinol-C₂ (CBN-C₂), cannabinodiol (CBND), cannabinodivarine (CBVD), cannabitriol (CBT), cannabitriolvarine (CBTV), déhydrocannabifurane (DCBF), cannabifurane, cannabicitrane (CBT), cannabiripsol (CBR), « 11-hydroxytétrahydrocannabinol » (11-OH-THC), « 11-nor-9-carboxy-tétrahydrocannabinol » (THC-COOH), et les dérivés, analogues, mélanges et combinaisons de ceuxci.

10. Conjugué cannabinoïde-acide hyaluronique selon la revendication 1, dans lequel le degré de réticulation est compris entre 5 % et 50 % des groupes de réticulation disponibles.

11. Conjugué cannabinoïde-acide hyaluronique selon la revendication 10, dans lequel le degré de réticulation est compris entre 10 % et 40 % des groupes de réticulation disponibles.

12. Conjugué cannabinoïde-acide hyaluronique selon la revendication 11, dans lequel le degré de réticulation est compris entre 20 % et 40 % des groupes de réticulation disponibles.

13. Composé ayant une formule de composé I : 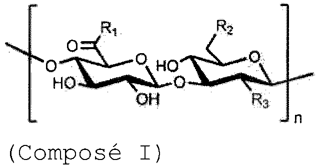
dans lequel : n = un motif répétitif du composé ;
R₁ = -ONa, -OH, ou un cannabinoïde lié par l'intermédiaire d'un espaceur ;
R₂ = -OH ou un cannabinoïde lié par l'intermédiaire d'un espaceur, dans lequel, si le cannabinoïde est CBD, l'espaceur n'est pas O ; et
R₃ = -NHCOCH₃ ou un cannabinoïde lié par l'intermédiaire d'un espaceur ;
et dans lequel au moins l'un de R₁, R₂ ou R₃ est un cannabinoïde lié par l'intermédiaire d'un espaceur, indépendamment dans un ou plusieurs des motifs répétitifs ; et
dans lequel un ou plusieurs des cannabinoïdes sont un agent de réticulation réticulant de manière covalente le squelette polysaccharidique.

14. Composé selon la revendication 13, dans lequel l'espaceur est choisi dans le groupe constitué par : O ; une chaîne alkyle ou une chaîne carbonée insaturée, linéaire ou ramifiée, substituée ou non ; C=O ; O=C-C=O ; O=C-(CH₂)nC=O où n est un nombre entier quelconque ; O=C-(CR₂)nC=O où R représente indépendamment H, un groupe alkyle, halogénure, CN, NH2, OH ou COOH ; NH-CO ; NH-alkyle; alkyl-C=O substitués ou non substitués, alkyl-NH-C=O, alcényl-C=O, alcényl NH-C=O, et alcynyl-C=O; des structures cycloalkyle, cycloalcényle et hétérocycliques ; des dérivés d'azole ; et triazole, facultativement substitué par des groupes alkyle, carbonyle ou alcoxy.

15. Composé selon la revendication 14, dans lequel l'espaceur est choisi dans le groupe constitué par L1, L2, L3 et L4 : 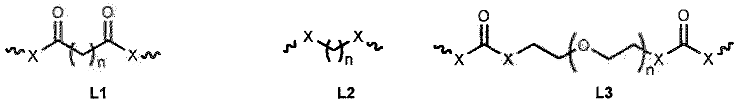 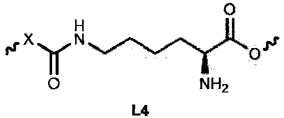 dans lequel, x = S, O ou NH ; et, pour L1 et L2, n = 1 à 10, et pour L3, n = 1 à 40.

16. Composé selon la revendication 15, dans lequel un deuxième cannabinoïde est lié au moyen d'un espaceur à l'un ou R₁, R₂ ou R₃, indépendamment dans un ou plusieurs motifs répétitifs.

17. Composé selon la revendication 13, dans lequel, dans un ou plusieurs motifs répétitifs :
R₁ = -ONa ou -OH ;
R₂ = -OH ; et
R₃=

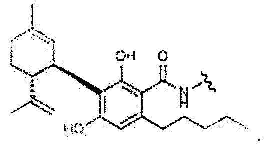

18. Composé selon la revendication 13, dans lequel, dans un ou plusieurs motifs répétitifs :
R₁ = -ONa ou -OH ;
R₃= 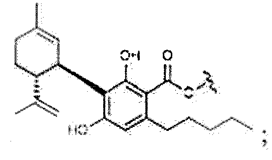 et
R₃ = -NHCOCH₃.

19. Composé selon la revendication 13, dans lequel, dans un ou plusieurs motifs répétitifs :
R₁= 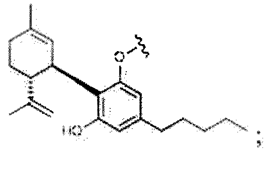
R₂ = -OH ; et
R₃ = -NHCOCH₃.

20. Composé selon la revendication 13, dans lequel, dans un ou plusieurs motifs répétitifs :
R₁ = -ONa ou -OH ; et
R₂= 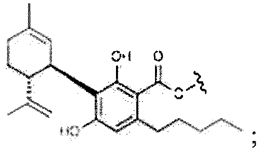 et
R₃=

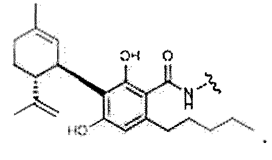

21. Composé selon la revendication 13, dans lequel, dans un ou plusieurs motifs répétitifs :
R₁ = -ONa ou -OH ;
R₂= 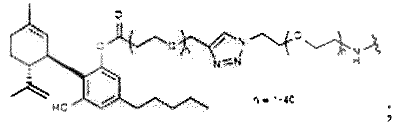 et
R₃ = -NHCOCH₃.

22. Composé selon la revendication 13, dans lequel, dans un ou plusieurs motifs répétitifs :
R₁ = -ONa ou -OH ;
R₂ = -OH ; et
R₃=

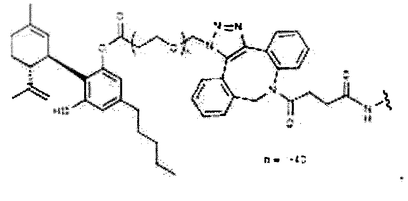

23. Composé selon la revendication 13, dans lequel, dans un ou plusieurs motifs répétitifs :
R₁ = -ONa ou -OH ;
R₂= 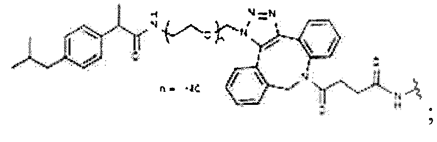 et
R₃=

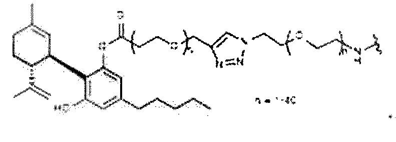

24. Composé selon la revendication 13, dans lequel, dans un ou plusieurs motifs répétitifs :
R₁ = -ONa ou -OH ;
R₂= 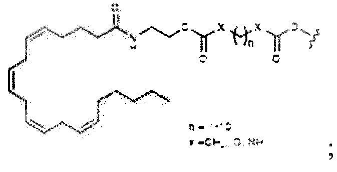 et
R₃=

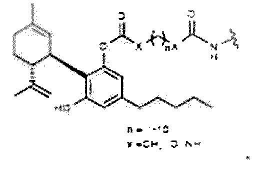

25. Composé selon la revendication 13, dans lequel, dans un ou plusieurs motifs répétitifs :
R₁ = -ONa ou -OH ;
R₂= 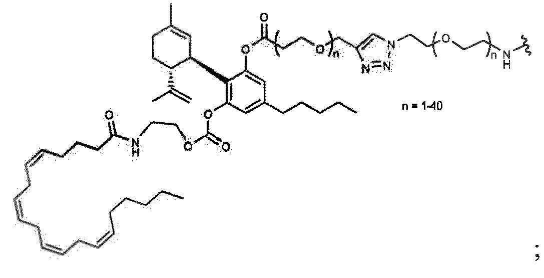 et
R₃ = -NHCOCH₃.

26. Composé selon la revendication 13, dans lequel, dans un ou plusieurs motifs répétitifs :
R₁ = -ONa ou -OH ;
R₂ = -OH ; et
R₃=

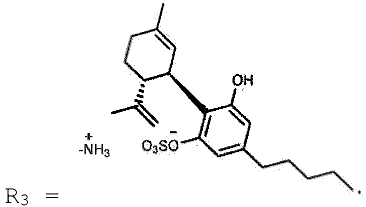

27. Composé selon la revendication 13, dans lequel, dans un ou plusieurs motifs répétitifs :
R₁ = -ONa ou -OH ;
R₂ = -OH ; et
R₃=

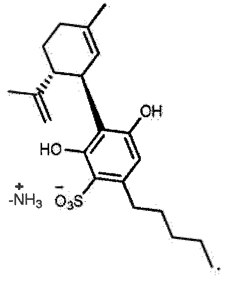

28. Composé selon la revendication 13, dans lequel plus d'un parmi R₁, R₂ ou R₃ est un cannabinoïde lié au moyen d'un espaceur, indépendamment dans un ou plusieurs motifs répétitifs.

29. Composé ayant une formule de composé II : 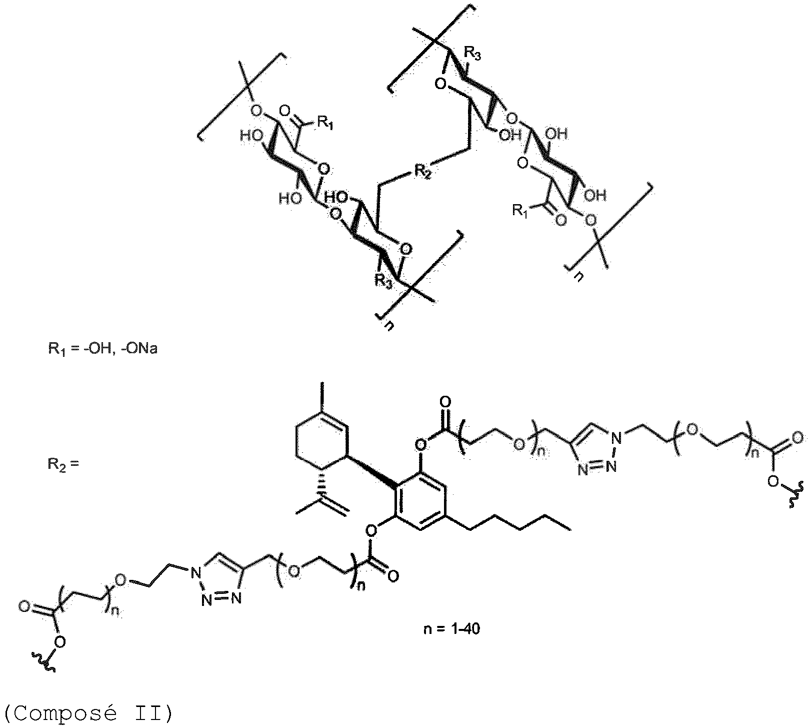
dans lequel : n = un motif répétitif du composé ; et
R₃ = -NHCOCH₃ ou un cannabinoïde lié par l'intermédiaire d'un espaceur ;
et dans lequel R₃ est un cannabinoïde lié au moyen d'un espaceur, indépendamment dans un ou plusieurs motifs répétitifs.

30. Composé selon la revendication 29, dans lequel R₃ représente

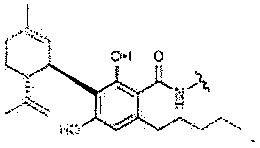

31. Composé selon la revendication 29, dans lequel R₃ représente

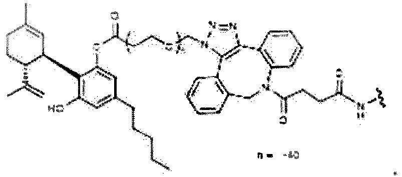

32. Composé selon la revendication 29, dans lequel R₃ représente

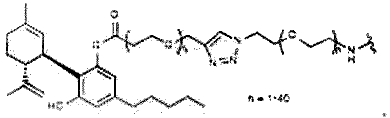

33. Composé selon la revendication 29, dans lequel R₃ représente

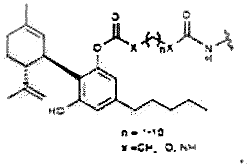

34. Composé selon la revendication 29, dans lequel R₃ représente

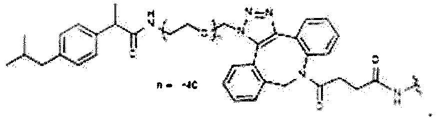

35. Composé selon la revendication 29, dans lequel R₃ représente

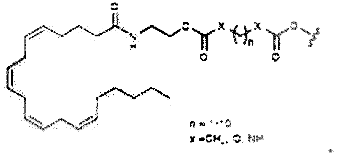

36. Composé selon la revendication 29, dans lequel R₃ représente

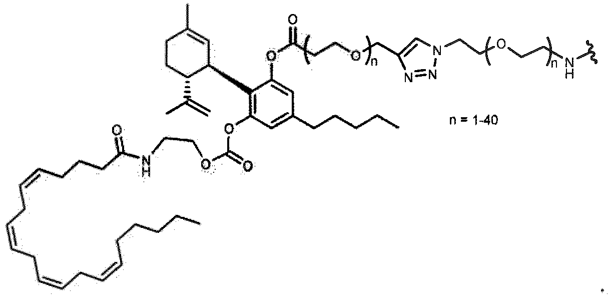

37. Formulation pharmaceutique sous forme de gouttes ophtalmiques pour le traitement du glaucome, comprenant un conjugué cannabinoïde-acide hyaluronique selon la revendication 1.

38. Formulation pharmaceutique selon la revendication 37, comprenant : le conjugué cannabinoïde-acide hyaluronique en une quantité comprise entre 0,0015 % p/p et 0,5 % p/p ; de la gomme arabique ou de la gomme xanthane en une quantité inférieure à 0,6 % p/p ; de l'acide borique en une quantité de 0,5 % p/p ; du chlorure de sodium en une quantité de 0,9 % p/p ; du sulfate de zinc en une quantité de 0,22 % p/p ; du citrate de sodium en une quantité de 0,2 % p/p ; du chlorure de benzalkonium en une quantité de 0,01 % p/p.

39. Formulation pharmaceutique sous forme injectable pour le traitement de l'arthrose, comprenant un conjugué cannabinoïde-acide hyaluronique selon la revendication 1.

40. Formulation topique pour le traitement de troubles cutanés, comprenant un conjugué cannabinoïde-acide hyaluronique selon la revendication 1.

41. Formulation topique selon la revendication 40, comprenant : le conjugué cannabinoïde-acide hyaluronique en une quantité comprise entre 0,5 % et 1 % p/p, de l'alcool cétylique en une quantité de 3,5 % p/p, de l'acide stéarique en une quantité de 4,75 % p/p, de la glycérine en une quantité de 3,5 % p/p, du propylèneglycol en une quantité de 4,0 % p/p, de l'huile de sésame en une quantité de 2,0 % p/p, de l'huile d'amande en une quantité de 3,0 % p/p, de l'huile de jojoba en une quantité de 0,5 % p/p, de l'huile d'argan en une quantité de 0,5 % p/p, de l'huile de noix de coco en une quantité de 0,5 % p/p, du monostéréate de polysorbitone en une quantité de 0,75 % p/p, du monoléate de polysorbate en une quantité de 1,75 % p/p et un extrait de plantes en une quantité inférieure à 0,65 %.

42. Formulation topique selon la revendication 41, dans laquelle l'extrait de plantes est un extrait d'une plante choisie dans le groupe constitué par : *Glycyrriza glabra, Curcuma longa, Psorolea corlifolia, Cassia tora, Areca catechu, Punica granatum, Embelica officinale, Centella asiatica, Cinnamon zeylanicum* et *Aloe vera.*

43. Formulation topique selon la revendication 41, dans laquelle l'extrait de plantes est un extrait de chanvre ou de marijuana.
